# EUROPEAN PATENT SPECIFICATION

(11) **EP 1 548 906 B1**
(45) Date of publication and mention of the grant of the patent: **23.02.2011**
(21) Application number: 03794079.8
(22) Date of filing: 11.08.2003
(51) Int. Cl.: H01T 23/00, H01T 19/04, A61L 9/22, B03C 3/02, B03C 3/40, B03C 3/60

(54) **ION GENERATING DEVICE, ION GENERATOR HAVING ION GENERATING DEVICE AND ELECTRIC APPARATUS HAVING ION GENERATOR**
IONENERZEUGUNGSEINRICHTUNG, IONENGENERATOR MIT EINER IONENERZEUGUNGSEINRICHTUNG UND ELEKTRISCHE VORRICHTUNG MIT DEM IONENGENERATOR
DISPOSITIF DE GÉNÉRATION D'IONS, GÉNÉRATEUR IONIQUE COMPORTANT UN DISPOSITIF DE GÉNÉRATION D'IONS ET DISPOSITIF ÉLECTRIQUE COMPORTANT UN GÉNÉRATEUR IONIQUE

(30) Priority: 04.09.2002 JP 2002259312; 19.09.2002 JP 2002273273
(43) Date of publication of application: 29.06.2005
(73) Proprietor: Sharp Kabushiki Kaisha, Osaka-shi, Osaka 545-8522 (JP)
(72) Inventor: SEKOGUCHI, Yoshinori, Kitakatsuragi-gun, Nara 639-0213 (JP); SUGIOKA, Ryoichi, Ibaraki-shi, Osaka 567-0887 (JP); URUSHISAKI, Masato, Osaka-shi, Osaka 547-0026 (JP)
(74) Representative: Brown, Kenneth Richard
(86) International application number: PCT/JP2003/010238
(87) International publication number: WO 2004/023615

(56) References cited:
- JP-A- 01 117 240
- JP-A- 08 240 968
- JP-A- 10 231 104
- JP-A- 63 053 883
- JP-A- 2001 042 598
- JP-A- 2001 093 648
- JP-A- 2001 093 650
- JP-A- 2002 117 957
- JP-U- 60 160 661
- JP-Y2- 2 511 953
- JP-Y2- 04 043 897

## Description

### Technical field

The present invention relates to an ion generating device that generates both positive and negative ions with which, for example, to kill or remove airborne bacteria present in air or to remove hazardous substances present in air. The present invention also relates to an ion generator provided with such an ion generating device, and to an electric apparatus provided with such an ion generator.

### Background art

In general, when many people are present in an air-tight room with little ventilation such as an office room or meeting room, air-polluting substances such as carbon dioxide exhaled by people, cigarette smoke, and dust increase and thereby cause negative ions present in air, which are believed to have an effect of relaxing humans, to decrease. In particular, cigarette smoke causes loss of a huge number of negative ions, reducing them to about one-half to one-fifth of their normal number. Thus, nowadays, various ion generators are commercially available for supplementing air with negative ions.

Without exception, however, conventional ion generators are of the type that generates only negative ions by a method relying on a high direct-current voltage. Thus, these generators indeed supplement air with negative ions, but do not actively remove airborne bacteria or hazardous substances present in air.

In recent years, therefore, ion generators that generate both positive and negative ions have been developed, and, today, air purifiers incorporating such ion generators are already commercially available. These air purifiers have a grid-shaped electrode and a plate-shaped electrode arranged so as to sandwich, from outside and inside respectively, a cylindrical glass tube used as a dielectric member.

When electric discharge is induced between these two electrodes, positive and negative ions are generated and are released into air. When these positive and negative ions attach to the surface of airborne bacteria or hazardous substances present in air, they produce, through a chemical reaction, radicals such as hydrogen oxide and hydroxyl radical. These radicals exert a decomposing effect, whereby airborne bacteria and hazardous substances present in air are removed.

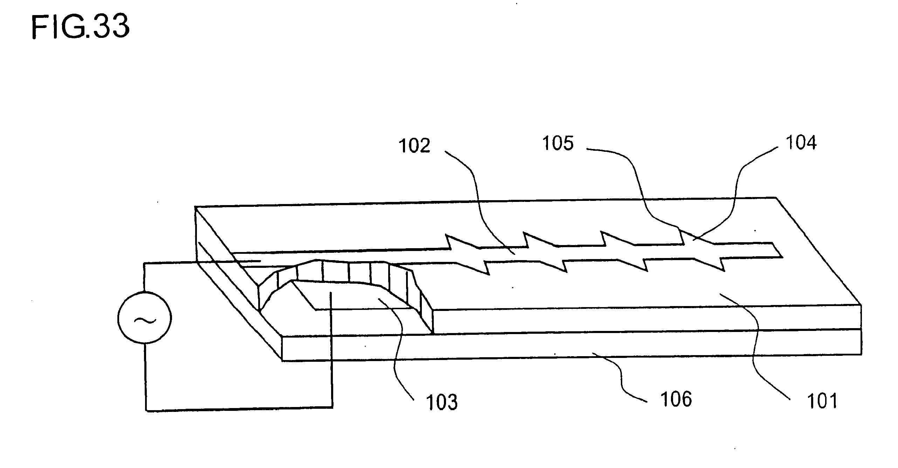

Fig. 33 shows an example of a conventionally proposed electric-field generator (see, for example, Japanese Patent Application Laid-Open No. H7-95477). This electric field generator has a linear discharge electrode 102 and a planar induction electrode 103 arranged so as to face each other across a dielectric substrate 101 lying between them. Moreover, a plurality of electric field concentration branch electrodes 104 are formed in the direction perpendicular to the direction of the longer sides of the linear discharge electrode 102. In this structure, when a high voltage is applied between the linear discharge electrode 102 and the planar induction electrode 103, corona discharge occurs near the linear discharge electrode 102, producing plasma containing both positive and negative ions on the dielectric substrate 101. Here, corona discharge starts at electric field concentration spots 105, i.e., the pointed ends of the electric field concentration branch electrodes 104, and then spreads over the entire linear discharge electrode 102. This helps to achieve reliable starting of electric discharge and to reduce the fluctuation of the discharge start voltage.

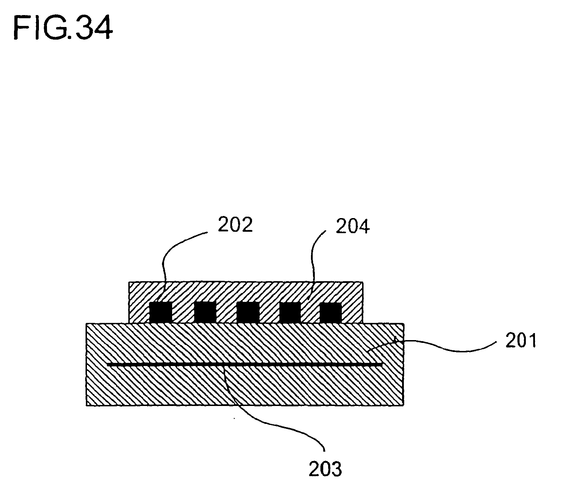

Fig. 34 shows another example of a conventionally proposed electric-field generator (see, for example, Japanese Patent Application Laid-Open No. H2-56793). This electric field generator has a plurality of linear discharge electrodes 202 formed on the surface of a dielectric substrate 201, and has a planar induction electrode 203, in the shape of a flat plate, formed so as to face the linear discharge electrodes 202 across the dielectric substrate 201 lying between them. Moreover, the linear discharge electrodes 202 is covered with a 1 to 40 µm thick alumina coating 204.

With this structure, even when electric discharge is induced between the linear discharge electrodes 202 and the planar induction electrode 203 in a high-humidity environment, and as a result NOx and hence nitric acid is generated, the alumina coating 204 is not corroded by nitric acid. This helps to enhance the durability of the electric-field generator.

However, in the air purifiers mentioned above as already commercially available, since the electrode arranged inside the dielectric member is an electrode in the shape of a plate, electric discharge occurs all over the plate-shaped electrode against the grid-shaped electrode. This wide discharge area results in producing, during electric discharge, loud discharge noise and an increased amount of ozone, exerting severe adverse effects on the human health.

The electric-field generator proposed in Japanese Patent Application Laid-Open No. H7-95477 mentioned above suffers from similar problems. Specifically, since the electrode arranged inside a dielectric member (between the dielectric substrate 101 and another dielectric substrate 106) is an electrode in the shape of plate (the planar induction electrode 103), electric discharge occurs all over the planar induction electrode 103 against the linear discharge electrode 102. This likewise results in producing loud discharge noise and an increased amount of ozone, exerting severe adverse effects on the human health.

In the electric-field generator proposed in Japanese Patent Application Laid-Open No. H2-56793 mentioned above, the alumina coating 204, with which the linear discharge electrodes 202 is covered, is formed to be so thick as to be at a constant distance from the surface of the dielectric substrate 201, be it in an area where the linear discharge electrodes 202 is formed or elsewhere. Thus, the thickness of the alumina coating 204 as measured in an area where the linear discharge electrodes 202 is not formed is greater than the thickness of the linear discharge electrodes 202.

Thus, when electric discharge occurs between the linear discharge electrodes 202 and the planar induction electrode 203, and the alumina coating 204 undergoes dielectric polarization, the generated electric field exhibits poor concentration near the linear discharge electrodes 202. As a result, positive and negative ions are generated in greatly unequal numbers. Thus, unless the discharge voltage between the linear discharge electrodes 202 and the planar induction electrode 203 is raised, it is impossible to obtain the desired numbers of ions. Raising the discharge voltage, however, invites problems similar to those mentioned above, producing, during electric discharge, loud discharge noise and an increased amount of ozone, exerting severe adverse effects on the human health.

### Disclosure of the invention

The present invention aims at solving the problems discussed above, and it is an object of the present invention to provide an ion generating device, an ion generator, and an electric apparatus that permit reduction of discharge noise and that offer excellent safety.

It is another object of the present invention to provide an ion generating device, an ion generator, and an electric apparatus that that permit generation of positive and negative ions in more equal numbers and that permit reduction of power consumption. This is achieved with an ion generating device, according to the features of claim 1.

In an ion generating device according to the present invention, an induction electrode formed inside a dielectric member is formed so as to be bent within a plane facing a discharge electrode formed on the surface of the dielectric member. The induction electrode is bent, in a U, shape as seen in a plan view.

Forming the induction electrode in a bent shape in this way, as compared with forming it in the shape of a plate all over the area in which it is formed, helps to reduce the discharge area of the induction electrode. This in turn helps to keep the discharge noise produced by the ion generating device low, and to reduce the amount of ozone produced during electric discharge, making the ion generating device highly safe to use.

In particular, forming the discharge electrode in the shape of a grid with pointed-end portions formed so as to project from the grid into the eyes thereof causes the electric field to concentrate between the pointed-end portions of the discharge electrode and the induction electrode, making it easier for electric charge to occur between the induction electrode and the discharge electrode. Thus, even if the discharge voltage (i.e., the voltage applied to the induction and discharge electrodes) is made lower than conventionally used, it is still possible to generate both positive and negative ions in numbers sufficient to deactivate airborne bacteria present in air or to remove hazardous substances present in air. Here, the word "deactivate" is used to encompass killing, removing, or reducing airborne bacteria present in air and decomposing or removing viruses.

Lowering the discharge voltage helps to reduce the power consumed by the ion generating device, and thereby to save electric power.

Similar effects can be obtained also by forming the discharge electrode so that it has at least one linear elongate portion with projecting portions so formed as to project therefrom in a direction different from (for example, perpendicular to) the direction in which the elongate portion extends.

In an ion generating device according to the present invention, when electric discharge (for example, corona discharge near the discharge electrode) is induced by a potential difference between the discharge electrode and the induction electrode, dielectric polarization occurs on the surface of a protective layer formed on the dielectric member. Here, the surface potential of the protective layer as measured near the discharge electrode (for example, in a portion of the protective layer located on the discharge electrode) is polarized to the same polarity as the discharge electrode, whereas the surface potential of the protective layer as measured away from the discharge electrode (for example, in a portion of the protective layer located on the dielectric member in an area where the discharge electrode is not formed) is polarized to the polarity opposite to the discharge electrode.

Here, the lines of electric force that run between the discharge and induction electrodes are denser the closer to the discharge electrode. In a case where, as in the present invention, the thickness (t1) of the protective layer as measured in an area where the discharge electrode is not formed is smaller than the thickness (t2) of the discharge electrode, the potential on the surface of the protective layer polarizes at positions where the lines of electric force is denser than in a case where t1 is substantially equal to or greater than t2.

This helps to increase the degree (electric field intensity) in which the electric field concentrates near the discharge electrode, and thus makes it possible to stably generate both positive and negative ions near the discharge electrode. This makes it possible to generate positive and negative ions in more equal numbers, and thereby to keep a proper ion balance.

Moreover, as a result of the electric field intensity near the discharge electrode being increased, even if the potential difference between the discharge and induction electrodes (the line-to-line voltage) is lowered, through the electric discharge mentioned above, it is possible to generate the desired numbers of positive and negative ions. This helps to reduce the power consumed by the ion generating device. Moreover, even when the line-to-line voltage is lowered, it is possible to generate positive and negative ions with a proper balance. This in turn helps to keep the discharge noise produced by the ion generating device low, and to reduce the amount of ozone produced during electric discharge, making the ion generating device highly safe to use.

In an ion generating device according to the present invention, it is preferable that the dielectric member mentioned above is in the shape of a flat plate (planar). This permits both the discharge and induction electrodes formed on the dielectric member to be formed in the shape of a flat plate (including a grid shape and a U shape). This, as compared with forming the dielectric member in a cylindrical shape, helps to improve the productivity of the dielectric member and hence the productivity of the ion generating device.

By a method for manufacturing an ion generating device according to the present invention, the ion generating device is built by forming a protective layer on a first dielectric member so that the thickness of the protective layer as measured in an area where no discharge electrode is formed is smaller than the thickness of the protective layer and then bonding the first dielectric member to a second dielectric member having an induction electrode formed thereon. This helps to increase the electric field intensity obtained when electric discharge (for example, corona discharge near the discharge electrode) is induced by a potential difference between the two electrodes mentioned above. Thus, effects similar to those described above can be obtained.

The effects described above can be obtained also by building an ion generator with the above-described ion generating device according to the present invention combined with a voltage feeding means for applying a voltage between the discharge and induction electrodes of the ion generating device. The effects described above can be obtained also by building various electric apparatuses (for example, air controllers, refrigerators, vacuum cleaners, and the like) with the above-described ion generator combined with a releasing means for releasing the ions generated by the ion generator into air.

### Brief description of drawings

Fig. 1A is a plan view showing an outline of the structure of an ion generator according to the invention.
Fig. 1B is a side view showing the above ion generator as seen from one side thereof.
Fig. 1C is a side view showing the above ion generator as seen from the other side thereof.
Fig. 2 is a diagram illustrating an outline of the construction of a vacuum cleaner provided with the above ion generator.
Fig. 3 is a diagram illustrating an outline of the construction of a refrigerator provided with the above ion generator.
Fig. 4 is a plan view showing an outline of the structure of the induction electrode provided in the ion generating device used in the above ion generator.
Fig. 5 is a plan view showing an outline of the structure of the discharge electrode provided in the above ion generating device.
Fig. 6 is a plan view showing, with enlargement, part A shown in Fig. 1A.
Fig. 7 is a plan view showing an outline of the structure of an ion generating device provided with a discharge electrode structured differently from the above discharge electrode.
Fig. 8 is a plan view showing another example of the structure of the above ion generating device.
Fig. 9A is a diagram showing the results of experiments conducted, with the ion generating device structured as shown in Fig. 7, to see how the numbers of positive and negative ions generated and the amount of ozone generated vary as the line-to-line voltage is varied.
Fig. 9B is a diagram showing the results of experiments conducted, with the ion generating device structured as shown in Fig. 8, to see how the numbers of positive and negative ions generated and the amount of ozone generated vary as the line-to-line voltage is varied.
Fig. 10 is a graph showing the relationship between the numbers of positive and negative ions generated and the line-to-line voltage as observed in the ion generating device structured as shown in Figs. 7 and 8.
Fig. 11 is a graph showing the relationship between the concentration of the ozone generated and the line-to-line voltage as observed in the ion generating device structured as shown in Figs. 7 and 8.
Fig. 12 is a plan view showing another example of the structure of the above ion generating device.
Fig. 13 is a plan view showing another example of the structure of the above ion generating device.
Fig. 14A is a diagram showing the results of experiments conducted, with the ion generating device structured as shown in Fig. 12, to see how the numbers of positive and negative ions generated and the amount of ozone generated vary as the line-to-line voltage is varied.
Fig. 14B is a diagram showing the results of experiments conducted, with the ion generating device structured as shown in Fig. 13, to see how the numbers of positive and negative ions generated and the amount of ozone generated vary as the line-to-line voltage is varied.
Fig. 15 is a graph showing the relationship between the numbers of positive and negative ions generated and the line-to-line voltage as observed in the ion generating device structured as shown in Figs. 12 and 13.
Fig. 16 is a graph showing the relationship between the concentration of the ozone generated and the line-to-line voltage as observed in the ion generating device structured as shown in Figs. 12 and 13.
Fig. 17 is a plan view showing another example of the structure of the above ion generating device.
Fig. 18A is a diagram showing the results of experiments conducted, with the ion generating device structured as shown in Fig. 12, to see how the numbers of positive and negative ions generated and the amount of ozone generated vary as the line-to-line voltage is varied.
Fig. 18B is a diagram showing the results of experiments conducted, with the ion generating device structured as shown in Fig. 17, to see how the numbers of positive and negative ions generated and the amount of ozone generated vary as the line-to-line voltage is varied.
Fig. 19 is a graph showing the relationship between the numbers of positive and negative ions generated and the line-to-line voltage as observed in the ion generating device structured as shown in Figs. 12 and 17.
Fig. 20 is a graph showing the relationship between the concentration of the ozone generated and the line-to-line voltage as observed in the ion generating device structured as shown in Figs. 12 and 17.
Fig. 21 is a plan view showing another example of the structure of the above ion generating device.
Fig. 22 is a plan view showing another example of the structure of the above ion generating device.
Fig. 23A is a diagram showing the results of experiments conducted, with the ion generating device structured as shown in Fig. 21, to see how the numbers of positive and negative ions generated and the amount of ozone generated vary as the line-to-line voltage is varied.
Fig. 23B is a diagram showing the results of experiments conducted, with the ion generating device structured as shown in Fig. 22, to see how the numbers of positive and negative ions generated and the amount of ozone generated vary as the line-to-line voltage is varied.
Fig. 24 is a graph showing the relationship between the numbers of positive and negative ions generated and the line-to-line voltage as observed in the ion generating device structured as shown in Figs. 21 and 22.
Fig. 25 is a graph showing the relationship between the concentration of the ozone generated and the line-to-line voltage as observed in the ion generating device structured as shown in Figs. 21 and 22.
Fig. 26 is a diagram illustrating the relationship between the positions of the discharge and induction electrodes relative to each other and the discharge start voltage.
Fig. 27A is a diagram illustrating a state in which a pointed-end portion of the discharge electrode is far from overlapping the induction electrode.
Fig. 27B is a diagram illustrating a state in which a pointed-end portion of the discharge electrode hardly overlaps the induction electrode.
Fig. 27C is a diagram illustrating a state in which a pointed-end portion of the discharge electrode overlaps the induction electrode.
Fig. 28 is a diagram illustrating the relationship between the way air is blown onto the discharge electrode and the number of ions generated as a result.
Fig. 29 is a diagram illustrating the lines of electric force running from the discharge electrode to the induction electrode with a positive voltage applied to the discharge electrode and a negative voltage to the induction electrode as observed in a case where no coating layer is formed on the dielectric member of the ion generating device.
Fig. 30 is a diagram illustrating the lines of electric force running from the discharge electrode to the induction electrode with a positive voltage applied to the discharge electrode and a negative voltage to the induction electrode as observed in a case where a coating layer is formed on the dielectric member and in addition the thickness of the coaling layer in an area where the discharge electrode is not formed is smaller than the thickness of the discharge electrode.
Fig. 31 is a diagram illustrating the lines of electric force running from the discharge electrode to the induction electrode with a positive voltage applied to the discharge electrode and a negative voltage to the induction electrode as observed in a case where a coating layer is formed on the dielectric member and in addition the thickness of the coaling layer in an area where the discharge electrode is not formed is substantially equal to the thickness of the discharge electrode.
Fig. 32 is a diagram illustrating the lines of electric force running from the discharge electrode to the induction electrode with a positive voltage applied to the discharge electrode and a negative voltage to the induction electrode as observed in a case where a coating layer is formed on the dielectric member and in addition the thickness of the coaling layer in an area where the discharge electrode is not formed is greater than the thickness of the discharge electrode.
Fig. 33 is a diagram illustrating an outline of the structure of a conventional electric field generator.
Fig. 34 is a diagram illustrating an outline of another example of the structure of a conventional electric field generator.

### Best mode for carrying out the invention

### EMBODIMENT 1

An embodiment of the present invention will be described below with reference to the relevant drawings.

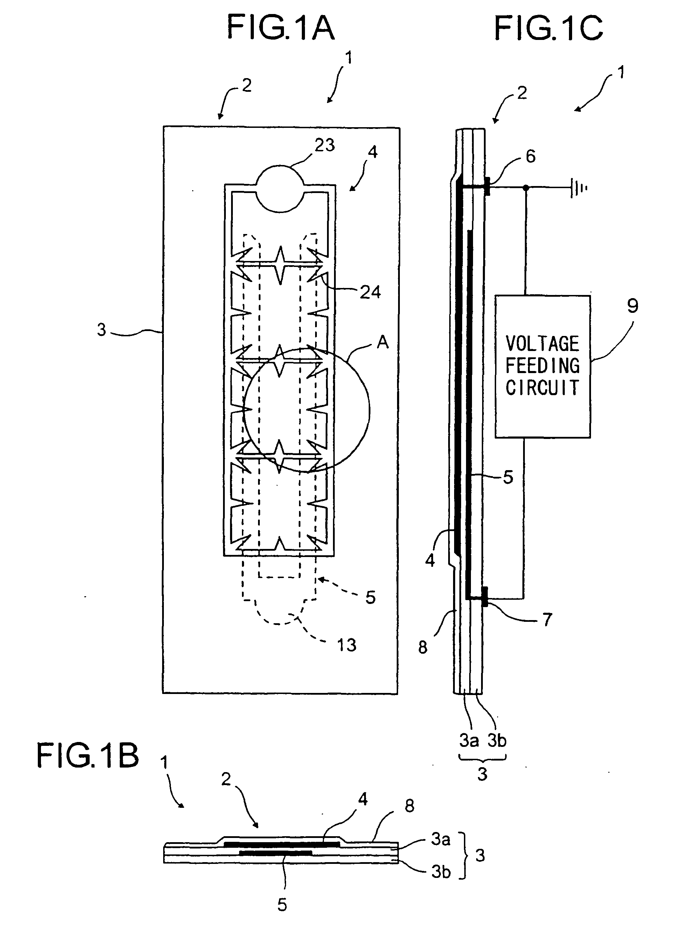

Fig. 1A is a plan view showing an outline of the structure of an ion generator 1 according to the invention. Fig. 1B is a side view of the ion generator 1 as seen from one side thereof. Fig. 1C is a side view of the ion generator 1 as seen from the other side thereof.

The ion generator 1 of the invention includes an ion generating device 2 and a voltage feeding circuit 9 (voltage feeding means).

The ion generating device 2 includes a dielectric member 3, a discharge electrode 4, an induction electrode 5, a discharge electrode contact 6, an induction electrode contact 7, a coating layer 8, and a resistor contact (not illustrated) to which a resistor is fused. Through electric discharge induced between the discharge electrode 4 and the induction electrode 5, the ion generating device 2 generates both positive and negative ions.

The dielectric member 3 is built by bonding together an upper dielectric member 3a and a lower dielectric member 3b, each in the shape of a rectangular parallelepiped, so as to have the shape of a flat plate as a whole. The dielectric member 3 may be formed of an organic material, in which case preferred materials are those highly resistant to oxidation, examples including resins such as polyimide and glass epoxy. The dielectric member 3 may be formed of an inorganic material, examples including ceramics such as high-purity alumina, crystallized glass, forsterite, and steatite.

From the viewpoint of resistance to corrosion, an inorganic material is preferred as the material for the dielectric member 3, and a ceramic is particularly suitable because of the ease it offers when the dielectric member 3 is shaped and also when the electrodes are formed as will be described later. Moreover, it is preferable that the insulation resistance between the discharge and induction electrodes 4 and 5 be uniform, and therefore, the more uniform the density of a material inside it, and thus the more uniform the insulation coefficient it gives to the dielectric member 3, the more suitable.

The dielectric member 3 may be formed in any other shape, for example in the shape of a circular plate, an elliptic plate, or a polygonal plate, or even in the shape of a circular column. From the viewpoint of productivity, however, it is preferable that the dielectric member 3 be formed in the shape of a flat plate (including the shape of a circular plate and of a rectangular parallelepiped) as shown in Figs. 1A, 1B, and 1C.

The discharge electrode 4 is formed on the surface of the dielectric member 3 (the upper dielectric member 3a) so as to be integral therewith. The discharge electrode 4 may be formed of any electrically conductive material, such as tungsten, so long as it does not become deformed, as by being melted, by electric discharge. It is preferable that the discharge electrode 4 have a uniform depth from the surface of the dielectric member 3 (in a case where the discharge electrode 4 is formed on the induction electrode 5 side of the surface of the dielectric member 3) or a uniform thickness (in a case where the discharge electrode 4 is formed so as to protrude from the surface of the dielectric member 3).

The discharge electrode 4 may be given a planar, grid-like, or linear shape. It is, however, preferable that, if possible, the discharge electrode 4 be given a grid-like or linear shape, i.e., a shape that permits easier concentration of the electric field against the induction electrode 5, because then, even if the voltage applied between the discharge and induction electrodes 4 and 5 is low, it is possible to induce electric discharge between the electrodes.

In this embodiment, the discharge electrode 4 is formed in the shape of a grid or comb teeth, of which a detailed description will be given later.

The induction electrode 5 is formed inside the dielectric member 3 (between the upper and lower dielectric members 3a and 3b), and is arranged so as to face the discharge electrode 4. This is because it is preferable that the insulation resistance between the discharge and induction electrodes 4 and 5 be uniform, and because it is preferable that the discharge and induction electrodes 4 and 5 be parallel to each other. This arrangement keeps the discharge and induction electrodes 4 and 5 at a constant distance (hereinafter referred to as the electrode-to-electrode distance) from each other. This ensures stable electric discharge between the discharge and induction electrodes 4 and 5, and makes it possible to properly generate positive and negative ions.

Like the discharge electrode 4, the induction electrode 5 is formed of any electrically conductive material, such as tungsten.

In this embodiment, the induction electrode 5 is formed in the shape of U, of which a detailed description will be given later.

The discharge electrode contact 6 connects to the discharge electrode 4 via a connection terminal 23 formed on the same surface as the discharge electrode 4. A lead formed of copper wire has one end connected to the discharge electrode contact 6 and the other end connected to the voltage feeding circuit 9 so that the discharge electrode 4 conducts to the voltage feeding circuit 9. The other end of the lead may be grounded.

For easy connection of the lead, the discharge electrode contact 6 may be formed anywhere on the surface of the dielectric member 3. Considering, however, that the discharge electrode contact 6 is at the same potential as the discharge electrode 4, to ensure stable electric discharge, it is preferable to form the discharge electrode contact 6 on the surface of the dielectric member 3 so that the distance between the induction electrode 5 and the discharge electrode contact 6 is greater than the aforementioned electrode-to-electrode distance.

The induction electrode contact 7 connects to the induction electrode 5 via a connection terminal 13 formed on the same surface as the induction electrode 5. A lead formed of copper wire has one end connected to the induction electrode contact 7 and the other end connected to the voltage feeding circuit 9 so that the induction electrode 5 conducts to the voltage feeding circuit 9. The other end of the lead may be grounded.

For easy connection of the lead, the induction electrode contact 7 may be formed anywhere on the surface of the dielectric member 3. Considering, however, that the induction electrode contact 7 is at the same potential as the induction electrode 5, to ensure stable electric discharge, it is preferable to form the induction electrode contact 7 on the surface of the dielectric member 3 so that the distance between the discharge electrode 4 and the induction electrode contact 7 is greater than the aforementioned electrode-to-electrode distance.

To ensure more stable electric discharge, it is further preferable to form the discharge and induction electrode contacts 6 and 7 on the surface of the dielectric member 3 so that the distance between the discharge and induction electrode contacts 6 and 7 is greater than the aforementioned electrode-to-electrode distance.

It is further preferable to form both the discharge and induction electrode contacts 6 and 7 on a surface of the discharge electrode 4 other than the one (hereinafter referred to as the upper surface) on which the discharge electrode 4 is formed. This permits the surface of the dielectric member 3 on which the discharge electrode 4 is formed to be left free of wiring members such as leads. Thus, even in a case where air is blown, from a separately provided fan or otherwise, onto the surface where the discharge electrode 4 is formed, the stream of air can be prevented from being disturbed by leads. This ensures that the positive and negative ions generated at the discharge electrode 4 are carried in a stream of air so as to be released out of the ion generating device.

With the above considerations in mind, in this embodiment, both the discharge and induction electrode contacts 6 and 7 are formed on the surface (hereinafter referred to as the lower surface) of the dielectric member 3 opposite to the one on which the discharge electrode 4 is formed.

Moreover, in this embodiment, the connection terminal 13 and the induction electrode contact 7, which are connected to the induction electrode 5, are formed outside the area over which the induction and discharge electrodes 5 and 4 overlap each other. This surely prevents uneven electric discharge resulting from the electric field concentrating between the connection terminal 13 and the discharge electrode 4 at the start of electric discharge. That is, from immediately after the start of electric discharge, it is possible to achieve uniform electric discharge everywhere between the induction and discharge electrodes 5 and 4. This makes it possible to release stable numbers of ions.

Likewise, in this embodiment, the connection terminal 23 and the discharge electrode contact 6, which are connected to the discharge electrode 4, are formed outside the area over which the induction and discharge electrodes 5 and 4 overlap each other. This provides effects similar to those described above. Specifically, it is possible to surely prevent uneven electric discharge resulting from the electric field concentrating between the connection terminal 23 and the induction electrode 5 at the start of electric discharge, and thereby to release stable numbers of ions.

The coating layer 8 protects the discharge electrode 4 formed on the upper surface of the dielectric member 3, and is formed of, for example, alumina (aluminum oxide).

The voltage feeding circuit 9 feeds a voltage to at least one of the discharge and induction electrodes 4 and 5. For example, in a case where both the discharge and induction electrodes 4 and 5 are connected to the voltage feeding circuit 9, the voltage feeding circuit 9 applies voltages to both of them. In a case where the discharge electrode 4 is grounded so as to be kept at a ground potential while the induction electrode 5 is connected to the voltage feeding circuit 9, the voltage feeding circuit 9 applies a voltage only to the induction electrode 5. In a case where the induction electrode 5 is grounded so as to be kept at a ground potential while the discharge electrode 4 is connected to the voltage feeding circuit 9, the voltage feeding circuit 9 applies a voltage only to the discharge electrode 4.

In a case where there is provided only one ion generating device 2, to make the ion generating device 2 generate both positive and negative ions, the voltage that is applied between the discharge and induction electrodes 4 and 5 by the voltage feeding circuit 9 needs to be an alternating voltage. This alternating voltage is not limited to an alternating voltage having a sinusoidal waveform (hereinafter, an alternating voltage having a sinusoidal waveform will be referred to as an alternating-current voltage) as commonly used as commercially distributed electric power, but may be an alternating voltage having a square or other waveform.

In the structure described above, when the voltage feeding circuit 9 is operated so that a high alternating-current voltage is applied between the discharge and induction electrodes 4 and 5, corona discharge occurs near the discharge electrode 4. This ionizes the air around the discharge electrode 4, generating positive ions, for example H⁺(H₂O)*ₘ* (where *m* is a natural number), and negative ions, for example O₂⁻(H₂O)*ₙ* (*n* is a natural number). These positive and negative ions are then released out of the ion generating device.

When these positive and negative ions attach to the surface of airborne bacteria or hazardous substances present in air, they produce, through a chemical reaction, radicals such as hydrogen oxide (H₂O₂) and hydroxyl radical (•OH). These radicals exert a decomposing effect, whereby airborne bacteria or hazardous substances present in air are destroyed. These positive and negative ions have been confirmed to have also a deodorizing effect.

The ion generator 1 structured as described above finds wide application in various electric appliances. Examples of such electric appliances include: air controllers, air conditioners, dehumidifiers, humidifiers, air purifiers, refrigerators, fan heaters, microwave ovens, washer-dryers, vacuum cleaners, sterilizers, and so on. These electric appliances are typically installed in a room in a house, in a room in a building, in a sickroom or operation room in a hospital, in a car, in an aircraft, in a ship, in a storehouse, in a compartment in a refrigerator, etc.

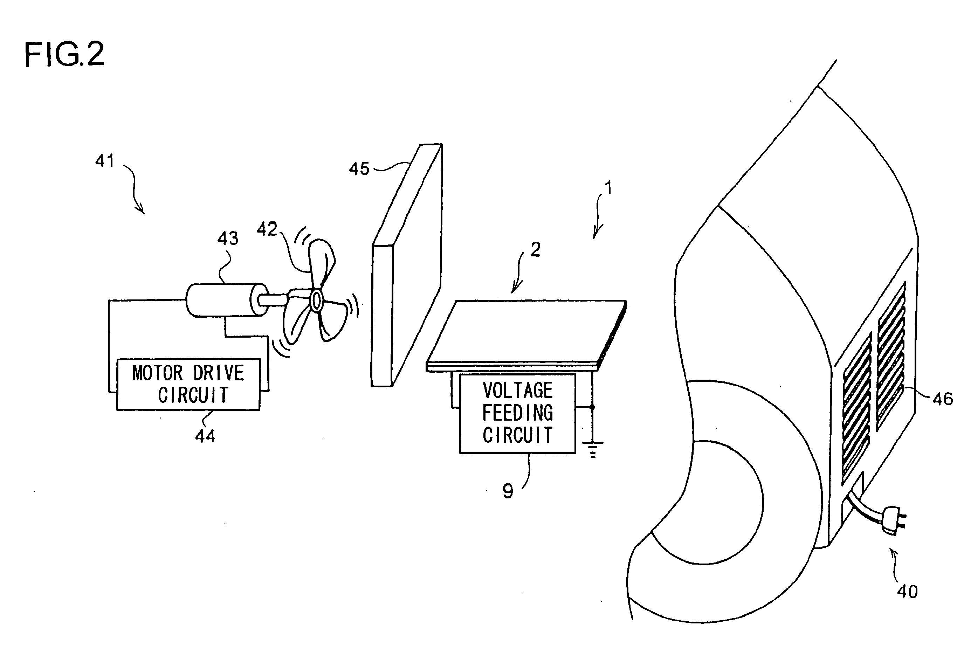

Fig. 2 shows an example in which the ion generator 1 of the invention is applied to a vacuum cleaner 40. The vacuum cleaner 40 is provided with the ion generator 1 in combination with a blower 41. The blower 41 serves as a releasing means for releasing the positive and negative ions generated by the ion generator 1 into air. The blower 41 is composed of a fan 42, a motor 43, and a motor drive circuit 44. Moreover, between the ion generator 1 and the blower 41 is arranged a filter 45 for removing fine particles.

In this construction, when the motor drive circuit 44 drives the motor 43, the fan 42 rotates, producing a stream of air. The positive and negative ions generated by the ion generator 1 are carried by this stream of air so as to be released out of the vacuum cleaner 40 through an exhaust port 46 thereof. Thus, it is possible to remove the dirt contained in the air in the room where the vacuum cleaner 40 is used and the dirt introduced in the air as the vacuum cleaner 40 is used.

Alternatively, the air sucked in by the vacuum cleaner 40 may be directly fed to the blower 41. In this case, the air sucked in is sent by the blower 41 to the ion generator 1, where the airborne substances and hazardous substances present in air are removed by the positive and negative ions generated by the ion generator 1. Thus, purified air is released out of the vacuum cleaner 40. With this construction, it is possible to effectively remove the dirt introduced into air as the vacuum cleaner 40 is used.

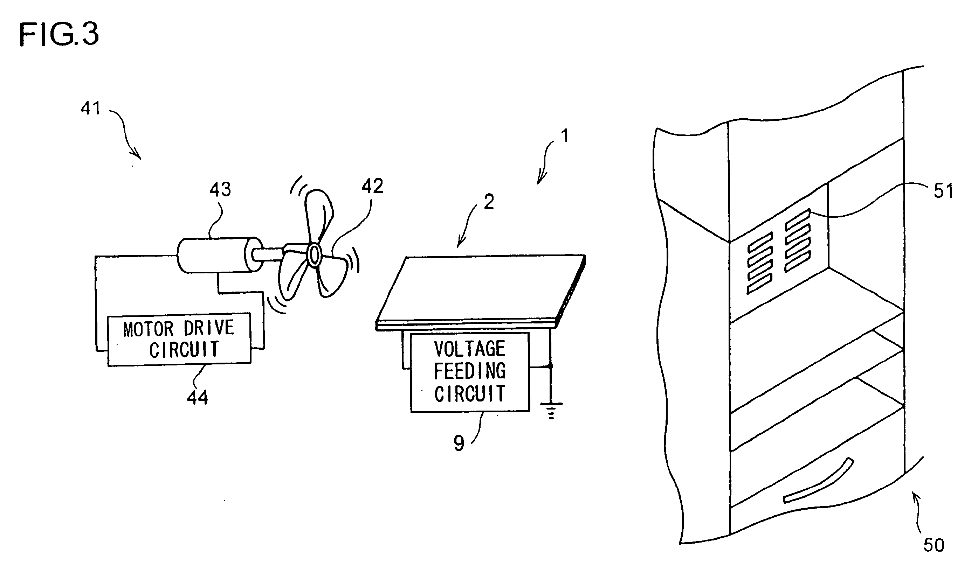

Fig. 3 shows an example in which the ion generator 1 of the invention is applied to a refrigerator 50. This refrigerator 50 is provided with the ion generator 1 in combination with a blower 41 that is built exactly like the one used in the vacuum cleaner 40.

In this construction, when the motor drive circuit 44 drives the motor 43, the fan 42 rotates, producing a stream of air. The positive and negative ions generated by the ion generator 1 are carried by this stream of air so as to be released into a space inside the refrigerator 50 through an air release port 51 thereof. Thus, it is possible to remove foul odors given off from the articles-to-be-cooled that are placed in the refrigerator 50.

Alternatively, the air inside the refrigerator 50 may be sucked in so as to be fed directly to the blower 41. In this case, air is purified before positive and negative ions are introduced into the refrigerator 50. Even then, purified air can be introduced into the refrigerator 50, and thus, just as in the case described above, it is possible to remove foul odors inside the refrigerator 50.

Next, as the distinctive feature of the present invention, the shape of the induction electrode 5 of the ion generating device 2 will be described in detail.

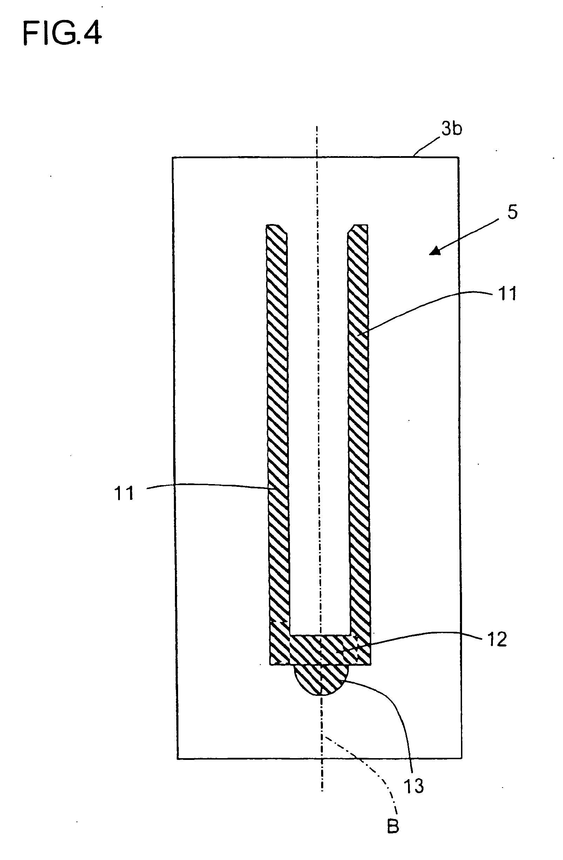

Fig. 4 is a plan view of the lower dielectric member 3b having the induction electrode 5 formed thereon. The induction electrode 5 is formed on the lower dielectric member 3b so as to be bent within a plane facing the discharge electrode 4 (see Fig. 1A).

More specifically, the induction electrode 5 is composed of two elongate portions 11 that extend parallel to the direction of the longer sides of the dielectric member 3 (the lower dielectric member 3b), a linking portion 12 that links together one ends of the elongate portions 11, and the previously described connection terminal 13, which is formed integrally with the linking portion 12. As a whole, the induction electrode 5 is formed in the shape of U as seen in a plan view. Moreover, the induction electrode 5 is so formed on the lower dielectric member 3b that the axis B about which the induction electrode 5 is line-symmetric coincides with the line that connects together the midpoints of the shorter sides of the lower dielectric member 3b.

The dimensions of these members are, for example, as follows. The elongate portions 11 are each formed to measure 1 mm in width (the length in the direction of the shorter sides of the lower dielectric member 3b) and 23.75 mm in length (the length in the direction of the longer sides of the lower dielectric member 3b). The linking portion 12 is formed to measure 1.5 mm in width (the length in the direction of the longer sides of the lower dielectric member 3b) and 2.5 mm in length (the length in the direction of the shorter sides of the lower dielectric member 3b). Thus, the elongate portions 11 are 2.5 mm apart from each other, and, since each of them measures 1 mm in width, the distance between the outer sides of the elongate portions 11 is 4.5 mm. The connection terminal 13 is formed to be semicircular and measure 1.5 mm in radius, and is so formed that the chord thereof coincides with a longer side of the linking portion 12.

Needless to say, the dimensions of the members specifically mentioned above are all merely examples, and are not meant as restrictions of any kind. This embodiment deals with a case where the induction electrode 5 is formed in a U shape; it is, however, also possible to form it in the shape of S or W.

Next, the shape of the discharge electrode 4 that is suitable for the ion generating device 2 provided with the induction electrode 5 structured as described above will be described.

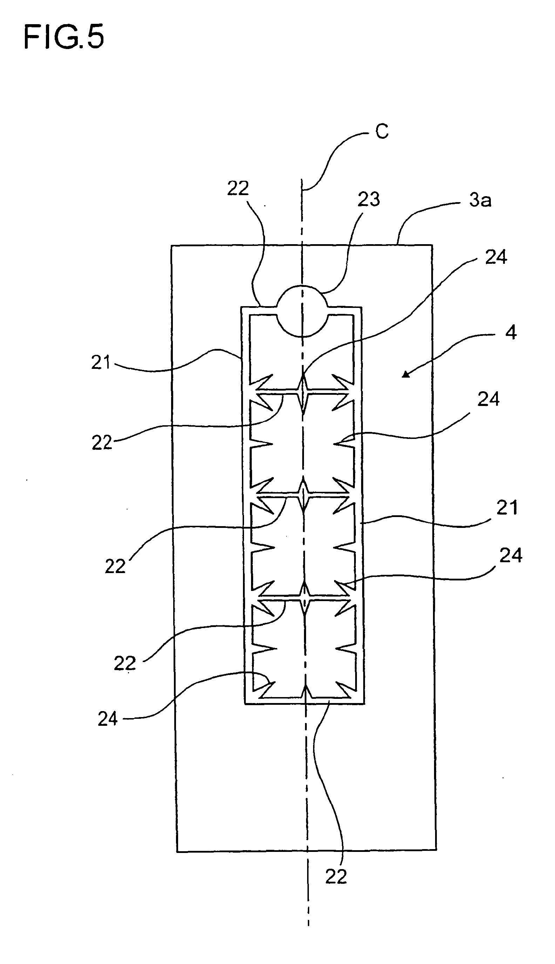

Fig. 5 is a plan view of the upper dielectric member 3a having the discharge electrode 4 formed thereon. The discharge electrode 4 is formed in the shape of a grid on the upper dielectric member 3a.

More specifically, the discharge electrode 4 has two elongate portions 21 that extend in the direction parallel to the longer sides of the upper dielectric member 3a and a plurality of linking portions 22 that are formed in the direction perpendicular to the direction in which the elongate portions 21 extend and that link together the two elongate portions 21 at different positions. Thus, each area enclosed by the two elongate portions 21 and two adjacent linking portions 22 form an eye of the grid.

In this embodiment, the discharge electrode 4 has four eyes of the grid formed one adjacent to the next in the direction of the longer sides of the upper dielectric member 3a. Of all the four eyes, three mutually adjacent ones are square in shape, and the last one is substantially rectangular in shape. A circular connection terminal 23 is formed integrally with the linking portion 22 that forms part of the perimeter of the discharge electrode 4, with the center of the connection terminal 23 located on the linking portion 22.

The distance between two linking portions 22 that are mutually adjacent to each other in the direction in which the elongate portions 21 extend is 6 mm. Thus, since the linking portions 22 have a width of 0.25 mm, the distance from the middle of one linking portion 22 to that of the adjacent one is 6.25 mm. In the following descriptions, this distance will be referred to as the pitch. Thus, the eyes of the grid formed as the discharge electrode 4 are formed with a pitch of 6.25 mm.

Needless to say, the dimensions of the members, the number of eyes of the grid, and the number of pointed-end portions 24 specifically mentioned above are all merely examples, and are not meant as restrictions of any kind.

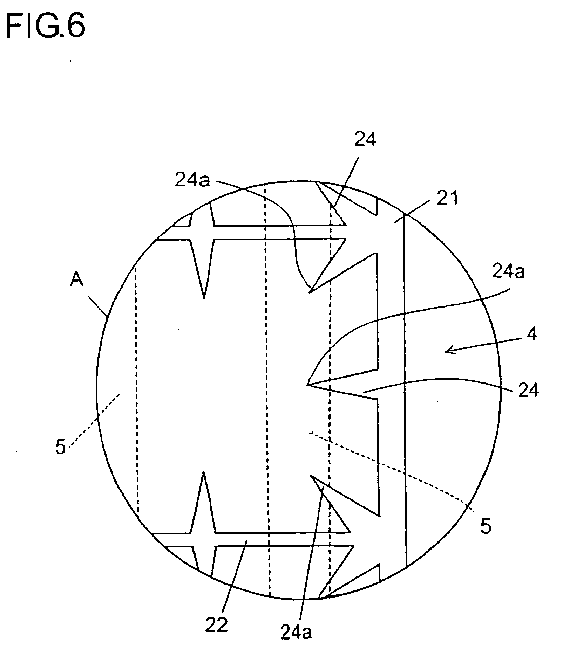

Moreover, in this embodiment, as shown in Fig. 6, the pointed-end portions 24 of the discharge electrode 4 are all so formed that the tip portions 24a thereof overlap the induction electrode 5 descried above. This structure is chosen on the basis of the results of experiments conducted in connection with Example 6, which will be described later. Thus, why this structure is chosen will be discussed later in connection with Example 6.

Moreover, the pointed-end portions 24 are so formed that the tip portions 24a thereof are located at equal distances (for example, 2 mm). This permits the areas over which the pointed-end portions 24 of the discharge electrode 4 overlap the induction electrode 5 to be spaced evenly, and this permits the electric field to concentrate uniformly between the discharge and induction electrodes 4 and 5. Thus, the ion generating device 2 generates positive and negative ions with a proper balance.

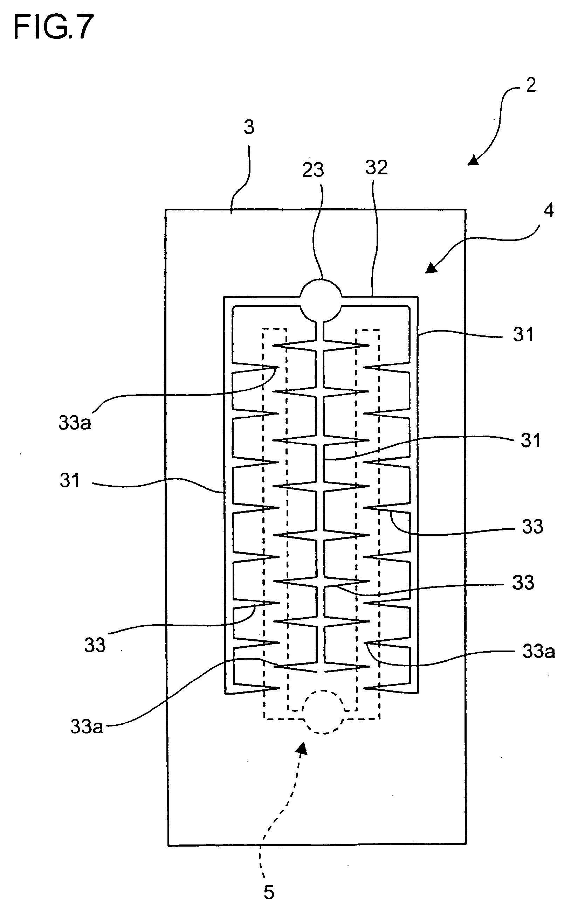

The discharge electrode 4 may be formed in, instead of the shape of a grid as described above, the shape of comb teeth. Fig. 7 is a plan view of an ion generating device 2 having a discharge electrode 4 formed in the shape of comb teeth. This induction electrode 5 has at least one linear elongate portion 31, specifically three of them in this embodiment. One ends of the elongate portions 31 are linked together by a linking portion 32, and a circular connection terminal 23 as described previously is formed integrally with the linking portion 32, with the center of the connection terminal 23 located on the linking portion 32.

The elongate portions 31 each have a plurality of projecting portions 33 formed so as to protrude therefrom within a plane facing the induction electrode 5 in a direction different from the direction in which the elongate portions 31 extend. In the following descriptions, the distance between two projecting portions 33 that are mutually adjacent in the direction in which the elongate portions 31 extend will be referred to as the pitch.

Also in this case, where the discharge electrode 4 is formed in the shape of comb teeth, the projecting portions 33 are so formed that the tip portions 33a thereof overlap the induction electrode 5 described above. This structure makes it easier for the electric field to concentrate between the projecting portions 33 of the discharge electrode 4 and the induction electrode 5, and thus makes it easier for electric discharge to occur between the two electrode even at a low voltage. This helps to surely reduce power consumption.

Next, the method for manufacturing the ion generating device 2 and the ion generator 1 according to the invention will be described.

First, a 0.45 mm thick sheet of high-purity alumina is cut into a predetermined size (for example, 15 mm wide × 37 mm long) to form two alumina base members of substantially the same size for use as an upper dielectric member 3a (a first dielectric member) and a lower dielectric member 3b (a second dielectric member). The alumina may be 90% or more pure, and specifically 92% pure alumina is used here.

Next, on the upper surface of the upper dielectric member 3a, a pattern of tungsten in the shape of a grid or comb teeth is screen-printed so that a discharge electrode 4 and a connection terminal 23 are formed integrally with the upper dielectric member 3a. On the other hand, on the top surface of the lower dielectric member 3b, a pattern of tungsten in the shape of U is screen-printed so that a induction electrode 5 is formed integrally with the lower dielectric member 3b, and, on the lower surface of the lower dielectric member 3b, a discharge electrode contact 6 and a induction electrode contact 7 are screen-printed.

Furthermore, on the surface of the upper dielectric member 3a, an alumina coating 8 with a thickness of, for example, 0.2 mm is formed so that the discharge electrode 4 is coated for electrical insulation. Here, the coating layer 8 is so formed that the thickness thereof in an area where the discharge electrode 4 is not formed is smaller than the thickness of the discharge electrode 4. This feature will be discussed later in connection with Example 2, which will be described later.

Then, with the lower surface of the upper dielectric member 3a and the upper surface of the lower dielectric member 3b put together, these members are press-fitted together, are then subjected to evacuation, and are then put in a furnace so as to be baked in a non-oxidizing environment at 1,400°C to 1,600°C. In this way, the ion generating device 2 according to the present invention can be easily manufactured.

Then, to permit a voltage to be applied between the discharge and induction electrodes 4 and 5 of the ion generating device 2, the ground potential of a separately provided circuit or the like (not illustrated) and the discharge electrode contact 6 are connected together by a lead, and the induction electrode contact 7 and the voltage feeding circuit 9 are connected together by a lead. In this way, the fabrication of the ion generator 1 is completed.

With a structure as described above where an induction electrode 5 is formed in a bent shape, as compared with a structure in which an induction electrode 5 is formed in the shape of a plate all over a rectangular area into which the induction electrode 5 fits, it is possible to reduce the discharge area of the induction electrode 5. This helps to keep the discharge noise produced by the ion generating device low, and to reduce the amount of hazardous ozone produced during electric discharge, making the ion generating device highly safe to use.

Below are presented, as Examples 1 to 7, the results of experiments conducted to evaluate the effects of an ion generating device 2 according to the present invention having a induction electrode 5 formed in the shape of U, with different shapes given to the discharge and induction electrodes 4 and 5 of the ion generating device 2.

### Example 1

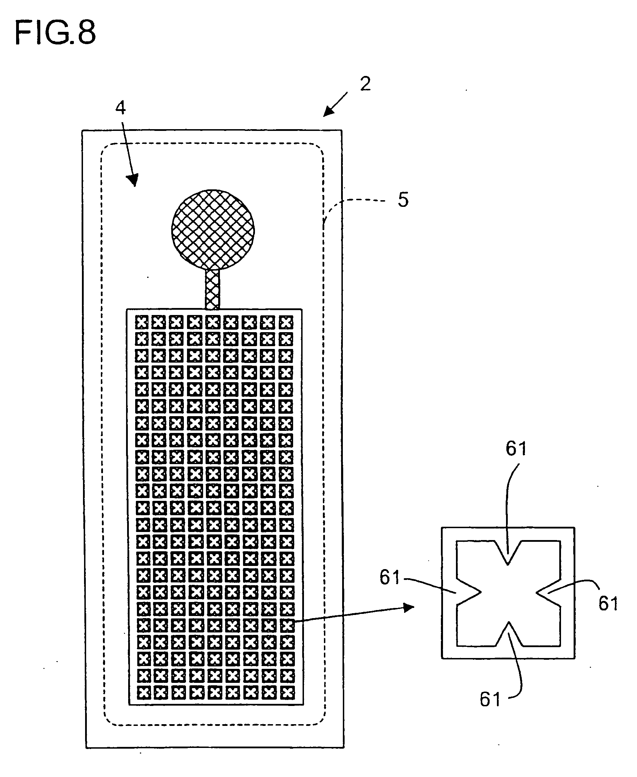

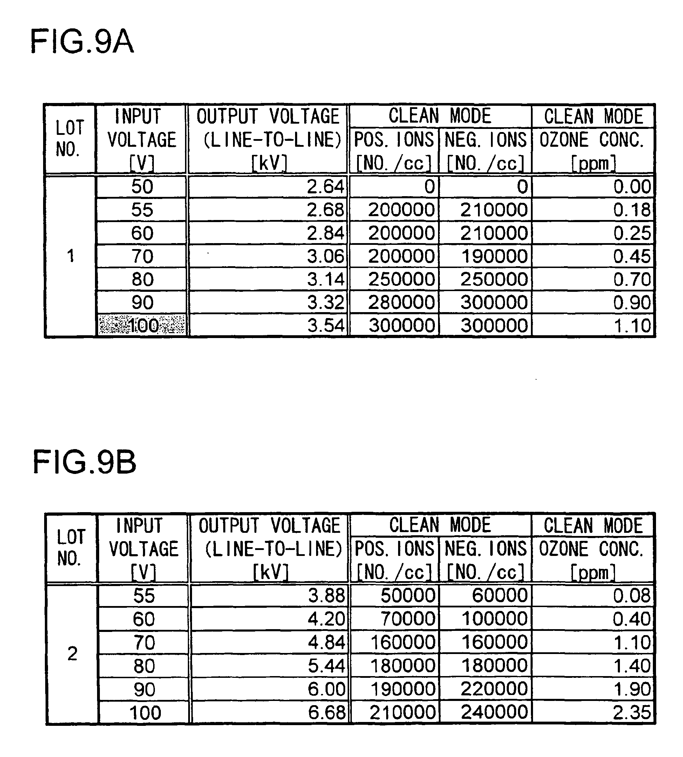

In this example, two types of ion generating device, shown in Figs. 7 and 8 respectively, were prepared, and while the voltage supplied to the voltage feeding circuit 9 (hereinafter also referred to as the input voltage) and the alternating-current voltage outputted therefrom (that is, the voltage between the discharge and induction electrodes 4 and 5, hereinafter also referred to as the line-to-line voltage) were varied, the numbers of positive and negative ions generated, the ozone generated, and the noise produced as a result were measured. The results with the different types of ion generating device are shown in Figs. 9A and 9B, respectively.

The input voltage was varied in the range from 50 V to 100 V. The line-to-line voltage was varied in the range from 2.64 kV to 6.68 kV, and the frequency of this voltage was about 40 kHz. The temperature was 23.3°C to 27.4°C, and the humidity was 38% to 40%.

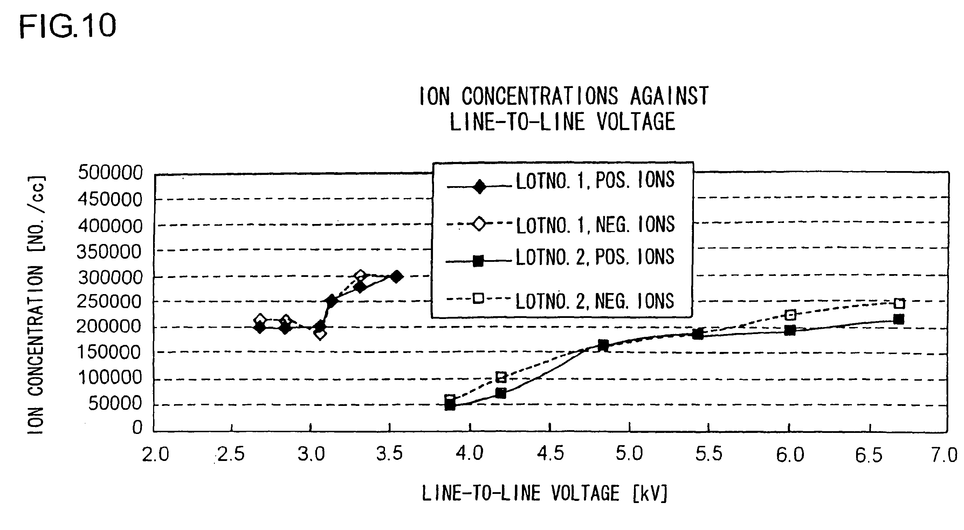

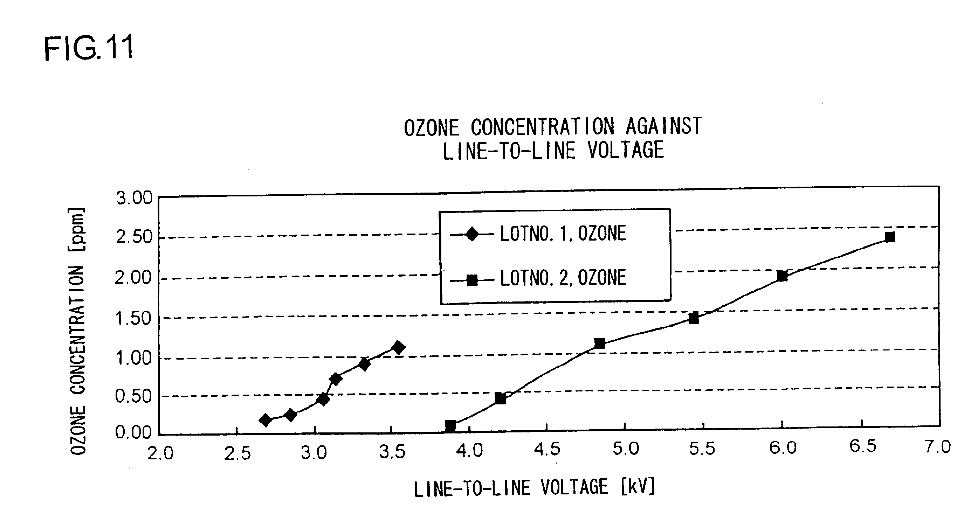

On the basis of the results shown in Figs. 9A and 9B, Fig. 10 shows a graphic representation of the relationship between the line-to-line voltage and the ion concentrations, and Fig. 11 shows a graphic representation of the relationship between the line-to-line voltage and the ozone concentration.

For the sake of simplicity, the ion generating device 2 shown Fig. 7 will be called Lot No. 1, and the ion generating device 2 shown in Fig. 8 will be called Lot No. 2.

Lot No. 1 has the induction electrode 5 formed in the shape of U, and has the discharge electrode 4 formed in the shape of comb teeth. In Lot No. 1, the discharge electrode 4 has 32 projecting portions 33, which are so formed that the tip portions 33a thereof overlap the U-shaped induction electrode 5. Accordingly, the number of intersections (overlaps) between the two electrodes is 32. Moreover, the projecting portions 33 are formed with a pitch of 3 mm.

On the other hand, Lot No. 2 has the induction electrode 5 formed in the shape of a flat plate, and has the discharge electrode 4 formed in the shape of a grid with dense eyes. In each eye of the grid, four pointed-end portions 61 are formed so as to protrude from the respective edges of the eye into the eye. Thus, in the entire discharge electrode 4, a total of 372 pointed-end portions 61 are formed. Accordingly, the number of intersections between the pointed-end portions 61 of the discharge electrode 4 and the induction electrode 5 is 372.

Figs. 9A, 9B, 10, and 11 show the following. Even with a lower line-to-line voltage, Lot No. 1 yields higher ion concentrations than Lot No. 2. Moreover, in Lot No. 1, the lower line-to-line voltage than in the Lot No. 2 results in a lower ozone concentration. Moreover, although when the input voltage is 100 V, Lot No. 1 and Lot No. 2 produce approximately the same level of discharge noise, when the input voltage is 50 V to 90 V, Lot No. 1 produces less discharge noise than Lot No. 2. It should be noted that, in the diagrams, hatching indicates an input voltage at which Lot No. 1 and Lot No. 2 produce the same level of discharge noise (this applies also to similar diagrams referred to later).

What is understood from this example is as follows. Forming the induction electrode 5 in the shape of U and reducing the number of intersections (the area of the overlaps) between the discharge and induction electrodes 4 and 5 help to reduce the amount of ozone generated and the level of discharge noise generated. Lowering the line-to-line voltage helps to reduce power consumption.

### Example 2

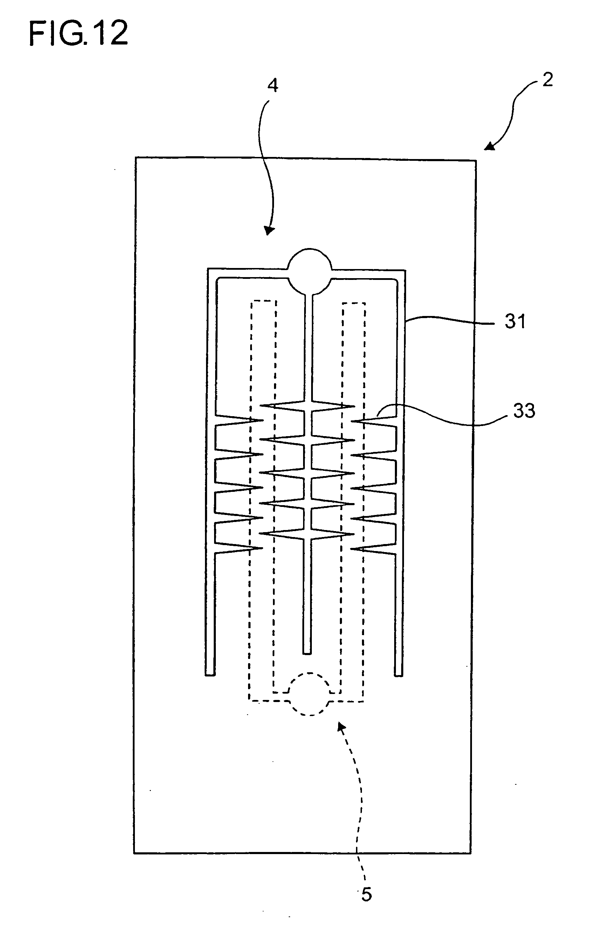

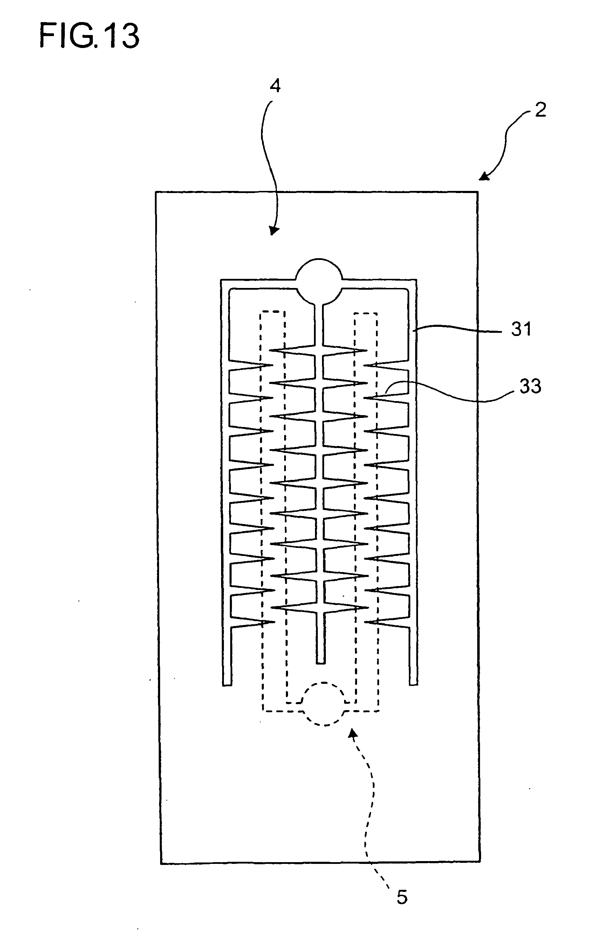

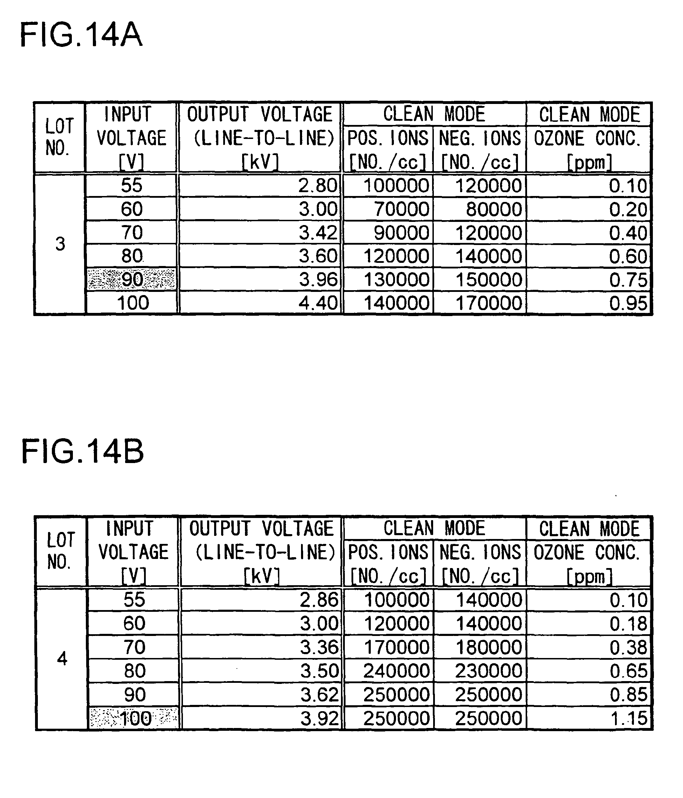

In this example, two types of ion generating device 2, shown in Figs. 12 and 13 respectively, were prepared, and, while the input voltage and the line-to-line voltage were varied, the numbers of ions generated, the amount of ozone generated, and the noise produced as a result were measured. The results with the different types of ion generating device are shown in Figs. 14A and 14B, respectively.

The input voltage was varied in the range from 55 V to 100 V. The line-to-line voltage was varied in the range from 2.80 kV to 4.40 kV, and the frequency of this voltage was about 40 kHz. The temperature was 24.6°C to 29.3°C, and the humidity was 36% to 38%.

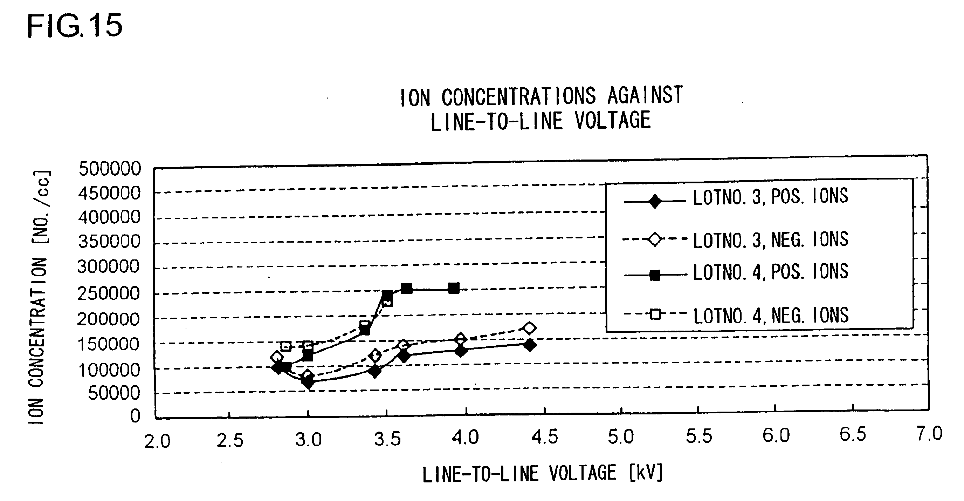

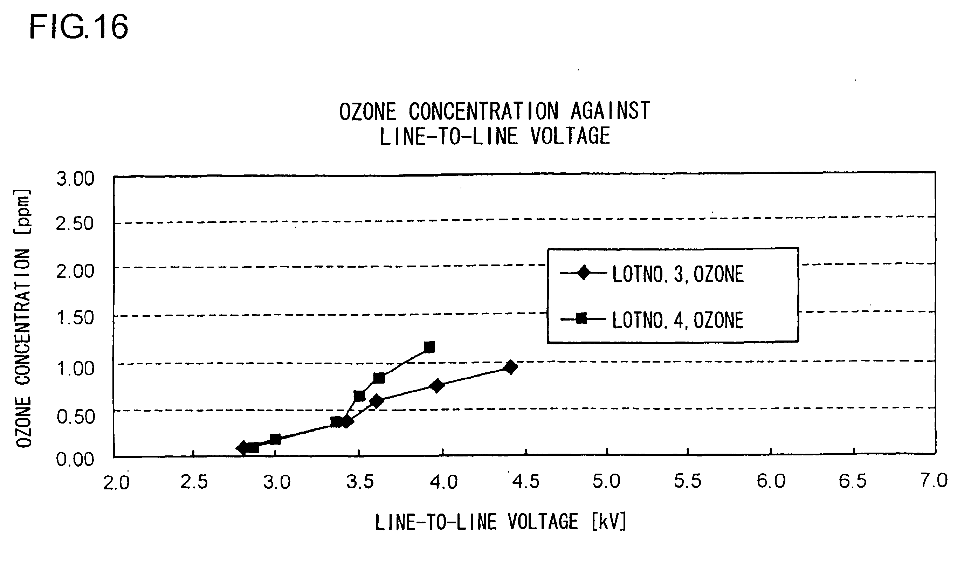

On the basis of the results shown in Figs. 14A and 14B, Fig. 15 shows a graphic representation of the relationship between the line-to-line voltage and the ion concentrations, and Fig. 16 shows a graphic representation of the relationship between the line-to-line voltage and the ozone concentration.

For the sake of simplicity, the ion generating device 2 shown Fig. 12 will be called Lot No. 3, and the ion generating device 2 shown in Fig. 13 will be called Lot No. 4.

Lot No. 3 and Lot No. 4 both have the discharge electrode 4 formed in the shape of comb teeth, and have the induction electrode 5 formed in the shape of U. In Lot No. 3, the discharge electrode 4 has 20 projecting portions 33, and the number of intersections (overlaps) between the discharge and induction electrodes 4 and 5 is twenty. By contrast, in Lot No. 4, the discharge electrode 4 has 36 projecting portions 33, and the number of intersections between the discharge and induction electrodes 4 and 5 is 36. In both Lot No. 3 and Lot No. 4, the projecting portions 33 are formed with a pitch of 2 mm.

Figs. 14A, 14B, 15, and 16 show the following. With the same line-to-line voltage, Lot No. 4 yields higher ion concentrations than Lot No. 3. Thus, the larger the number of projecting portions 33 the discharge electrode 4 has (the larger the number of intersections between the discharge and induction electrodes 4 and 5), the more the number of ions generated with the same line-to-line voltage. Moreover, with the same line-to-line voltage, Lot No. 4 yields a higher ozone concentration than Lot No. 3. Thus, the larger the number of projecting portions 33 the discharge electrode 4 has, the larger the amount of ozone generated.

When the input voltage is 90 V, Lot No. 3 produces substantially the same level of discharge noise as Lot No. 2 does with the same input voltage; when the input voltage is 55 V to 80 V, Lot No. 3 produces less noise than Lot No. 2 does with the same input voltage. On the other hand, when the input voltage is 100 V, Lot No. 4 produces substantially the same level of discharge noise as Lot No. 2 does with the same input voltage; when the input voltage is 55 V to 90 V, Lot No. 4 produces less noise than Lot No. 2 does with the same input voltage.

What is understood from this example is as follows. The smaller the number of projecting portions 33 the discharge electrode 4 has, the more effectively it is possible to reduce the amount of ozone generated and the discharge noise produced.

### Example 3

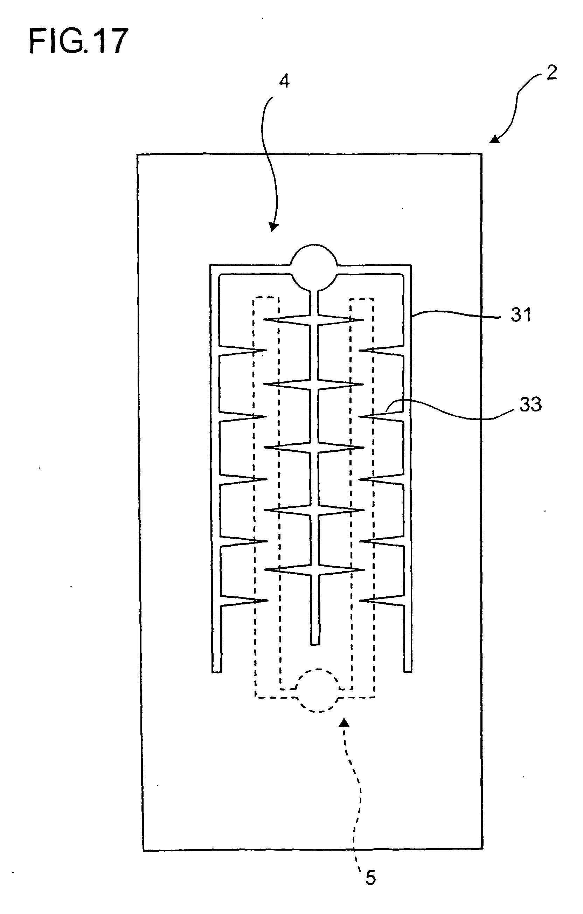

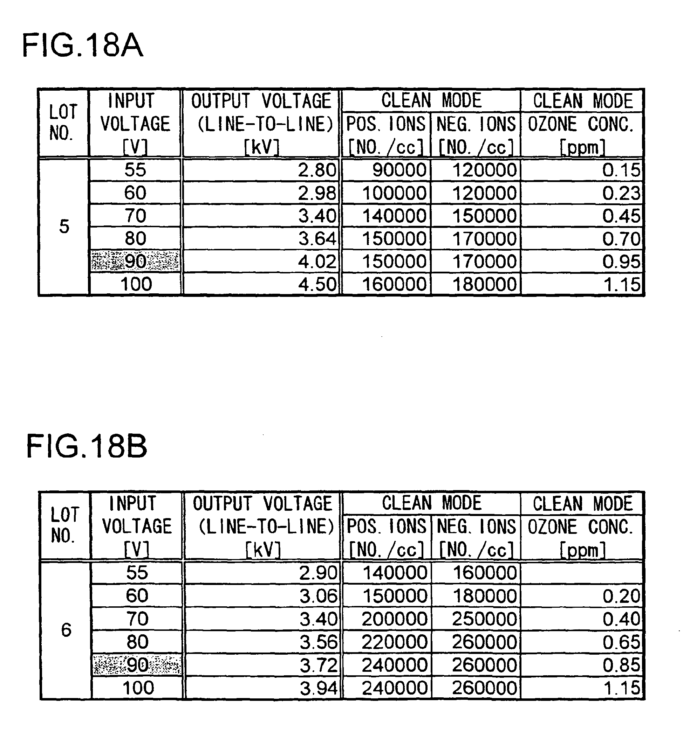

In this example, two types of ion generating device 2, shown in Figs. 12 and 17 respectively, were prepared, and, while the input voltage and the line-to-line voltage were varied, the numbers of ions generated, the amount of ozone generated, and the noise produced as a result were measured. The results with the different types of ion generating device are shown in Figs. 18A and 18B, respectively.

The input voltage was varied in the range from 55 V to 100 V. The line-to-line voltage was varied in the range from 2.80 kV to 4.50 kV, and the frequency of this voltage was about 40 kHz. The temperature was 24.4°C to 27.6°C, and the humidity was 28% to 29%. It should be noted that, in the diagrams, a blank cell indicates that no data was available because of unstable electric discharge.

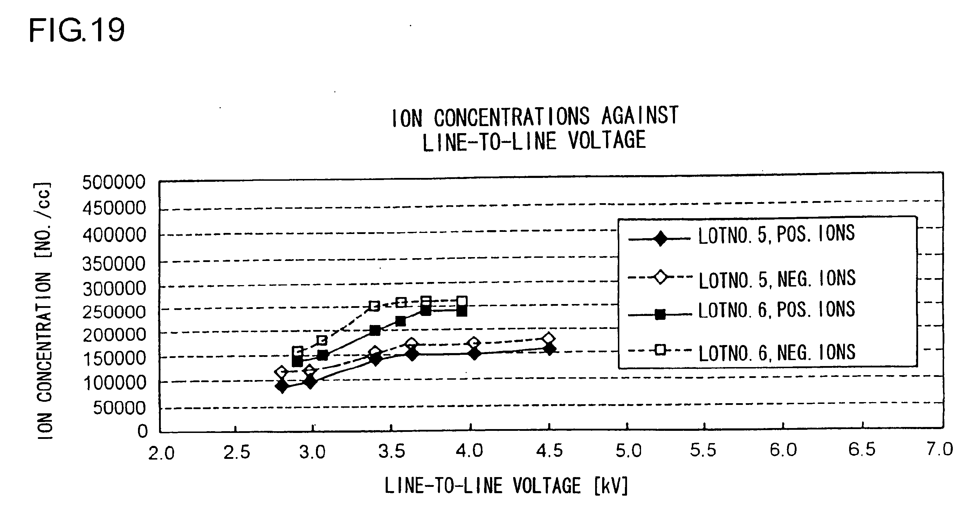

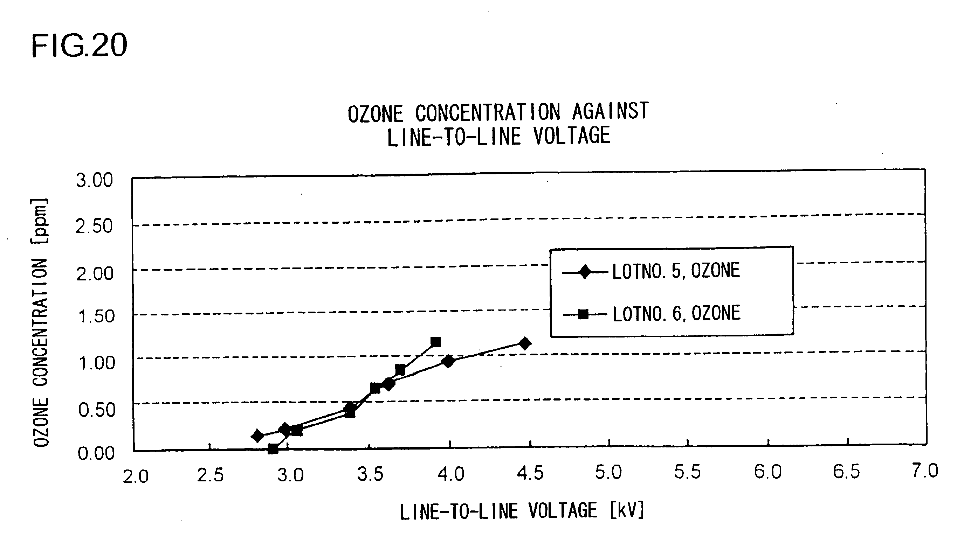

On the basis of the results shown in Figs. 18A and 18B, Fig. 19 shows a graphic representation of the relationship between the line-to-line voltage and the ion concentrations, and Fig. 20 shows a graphic representation of the relationship between the line-to-line voltage and the ozone concentration.

For the sake of simplicity, the ion generating device 2 shown Fig. 12 will be called Lot No. 5, and the ion generating device 2 shown in Fig. 17 will be called Lot No. 6.

Lot No. 5 and Lot No. 6 both have the discharge electrode 4 formed in the shape of comb teeth, and have the induction electrode 5 formed in the shape of U. Lot No. 5 has quite the same structure as Lot No. 3 of Example 2. Specifically, in Lot No. 5, the discharge electrode 4 has 20 projecting portions 33, and the number of intersections between the discharge and induction electrodes 4 and 5 is twenty, with the projecting portions 33 formed with a pitch of 2 mm. On the other hand, Lot No. 6 has quite the same structure as Lot No. 5 except that, in Lot No. 6, the motor drive circuit projecting portions 33 are formed with a pitch of 4 mm.

Despite Lot No. 5 having quite the same structure as Lot No. 3, the results shown in Fig. 18A in connection with Lot No. 5 are slightly different from those shown in Fig. 14A in connection with the Lot No. 3. These differences results from the differences in the environmental conditions (for example, temperature and humidity) under which the experiments were conducted with the different lots.

Figs. 18A, 18B, 19, and 20 show the following. With the same line-to-line voltage, Lot No. 6 yields higher ion concentrations than Lot No. 5. Thus, with a given line-to-line voltage, the greater the pitch with which the projecting portions 33 are formed in the discharge electrode 4 (the greater the distances between the projecting portions 33), the larger the number of ions generated. Moreover, with the same line-to-line voltage, Lot No. 5 and Lot No. 6 yield nearly the same ozone concentration.

When the input voltage is 90 V, Lot No. 5 and Lot No. 6 both produce approximately the same level of discharge noise as Lot No. 2 does with the same input voltage; when the input voltage is 55 V to 80 V, Lot No. 5 and Lot No. 6 produce less noise than Lot No. 2 does with the same input voltage.

What is understood from this example is as follows. With a given line-to-line voltage, the greater the pitch with which the projecting portions 33 are formed in the discharge electrode 4, the more efficiently it is possible to generate ions.

### Example 4

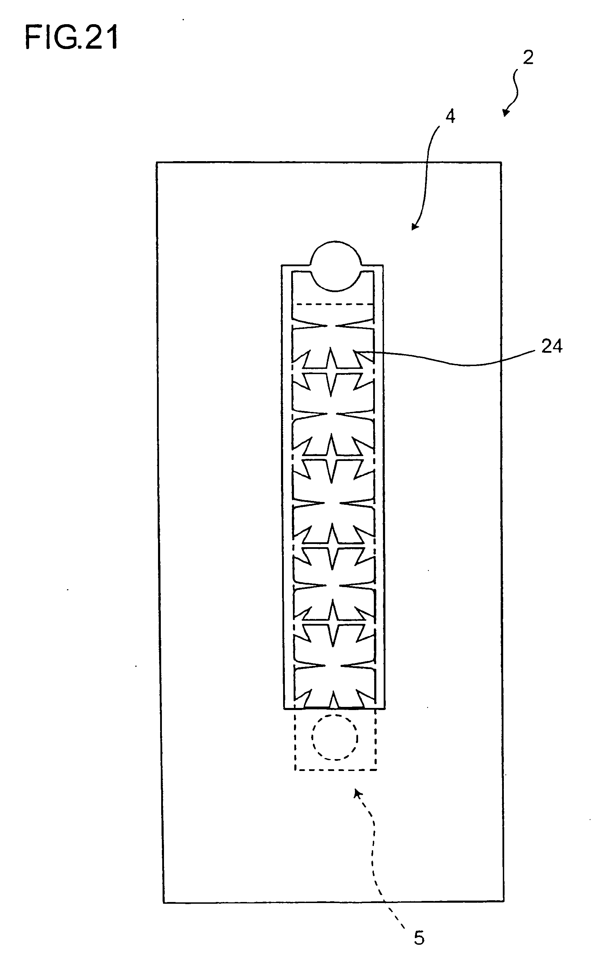

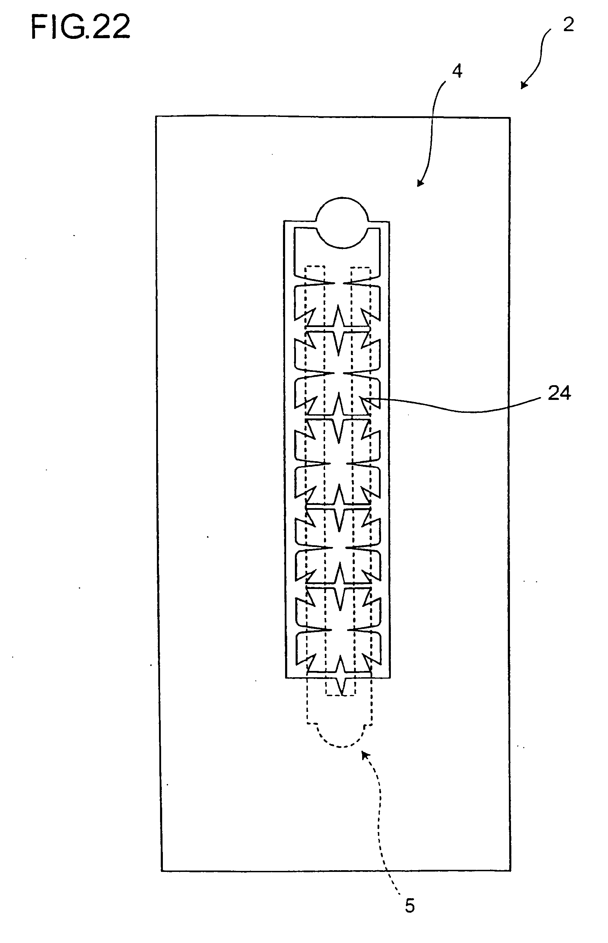

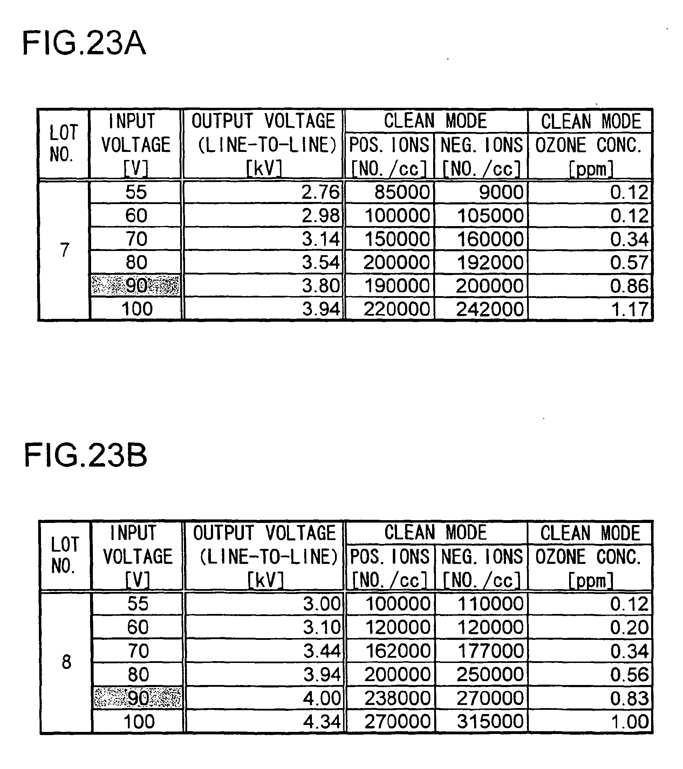

In this example, two types of ion generating device 2, shown in Figs. 21 and 22 respectively, were prepared, and, while the input voltage and the line-to-line voltage were varied, the numbers of ions generated, the amount of ozone generated, and the noise produced as a result were measured. The results with the different types of ion generating device are shown in Figs. 23A and 23B, respectively.

The input voltage was varied in the range from 55 V to 100 V. The line-to-line voltage was varied in the range from 2.76 kV to 4.34 kV, and the frequency of this voltage was about 40 kHz. The temperature was 24.9°C to 28.8°C, and the humidity was 40% to 44%.

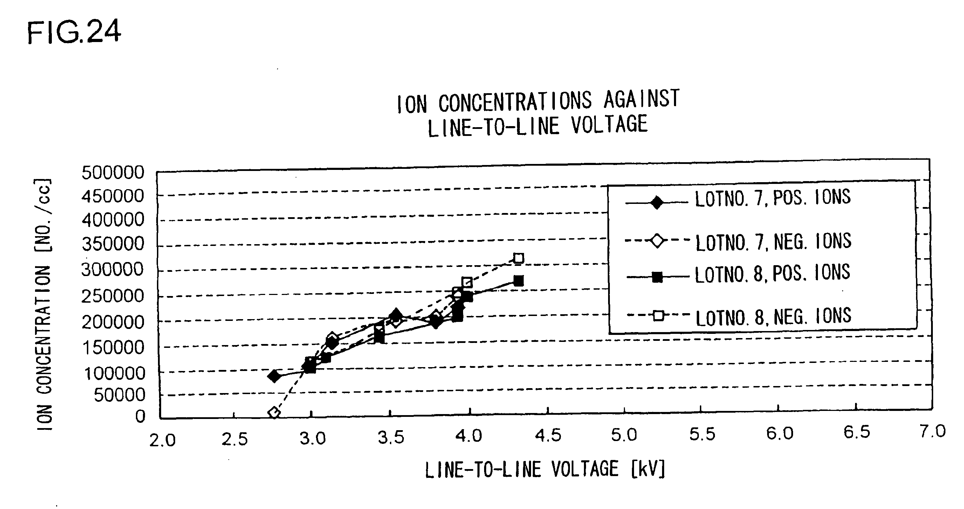

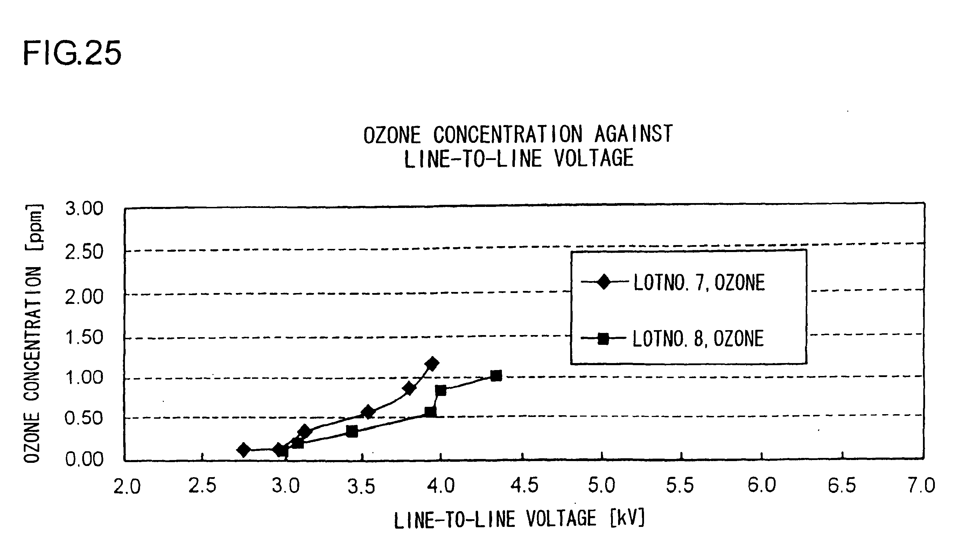

On the basis of the results shown in Figs. 23A and 23B, Fig. 24 shows a graphic representation of the relationship between the line-to-line voltage and the ion concentrations, and Fig. 25 shows a graphic representation of the relationship between the line-to-line voltage and the ozone concentration.

For the sake of simplicity, the ion generating device 2 shown Fig. 21 will be called Lot No. 7, and the ion generating device 2 shown in Fig. 22 will be called Lot No. 8.

Lot No. 7 has the induction electrode 5 formed in the shape of a flat plate as conventionally practiced, and has the discharge electrode 4 formed in the shape of a grid with five eyes arranged in a row, with 37 pointed-end portions 24 formed therein. Thus, in Lot No. 7, the number of overlaps between the pointed-end portions 24 of the discharge electrode 4 and the induction electrode 5 is 37, equal to the number of pointed-end portions 24 themselves.

On the other hand, Lot No. 8 has the same structure as Lot No. 7, except that the Lot No. 8 has the induction electrode 5 formed in the shape of U. Accordingly, also in Lot No. 8, the number of pointed-end portions 24 formed in the discharge electrode 4 is 37 as in Lot No. 7. However, in Lot No. 8, since the induction electrode 5 is formed in the shape of U, those pointed-end portions 24 located on the axis about which the discharge electrode 4 is line-symmetric do not overlap the induction electrode 5, and thus the number of overlaps between the tips of the pointed-end portions 24 of the discharge electrode 4 and the induction electrode 5 is 28, equal to the number (nine) of those pointed-end portions 24 subtracted from the total number of pointed-end portions 24.

Figs. 23A, 23B, 24, and 25 show the following. As the line-to-line voltage varies, the ion concentrations vary in largely the same manner in Lot No. 7 and in Lot No. 8; with the same line-to-line voltage, however, Lot No. 8 evidently yields a lower ozone concentration than Lot No. 7.

When the input voltage is 100 V, Lot No. 7 produces approximately the same level of discharge noise as Lot No. 2 does with the same input voltage; when the input voltage is 55 V to 90 V, Lot No. 7 produces less discharge noise than Lot No. 2 does with the same input voltage. On the other hand, when the input voltage is 90 V, Lot No. 8 produces approximately the same level of discharge noise as Lot No. 2 does with the same input voltage; when the input voltage is 55 V to 80 V, Lot No. 8 produces less discharge noise than Lot No. 2 does with the same input voltage.

What is understood from this example is as follows. Forming the induction electrode 5 in the shape of U rather than forming it in the shape of a flat plate, and reducing the number of intersections between the pointed-end portions 24 of the discharge electrode 4 and the induction electrode 5, helps reduce the amount of ozone generated and the discharge noise produced.

The results presented above in connection with Examples 1 to 4 show the following. Even with a reduced line-to-line voltage, forming the induction electrode 5 in the shape of U, reducing the number of intersections between the pointed-end portions 24 or projecting portions 33 of the discharge electrode 4 and the induction electrode 5, and increasing the pitch with which the pointed-end portions 24 or projecting portions 33 are formed make it possible to efficiently generate positive and negative ions. Moreover, reducing the line-to-line voltage helps to surely reduce the power consumed by the ion generating device, and also to surely reduce the ozone generated and the noise produced by the ion generating device.

Moreover, forming the induction electrode 5 in the shape of U, as compared with forming it in the shape of S or W, helps to surely reduce the discharge area of the induction electrode 5. This makes it possible to more surely obtain the effects described above.

### Example 5

In this example, how uniform the electric field between the discharge and induction electrodes 4 and 5 was and how it related to the ion balance (the balance between the numbers of positive and negative ions) were investigated. For these purposes, two types of ion generating device 2 having the discharge electrode 4 formed in different grid patterns were prepared, and, with each of them, whether a proper ion balance was obtained or not was checked.

Here, one ion generating device 2 has, as shown in Fig. 5, the discharge electrode 4 formed in the shape of a grid with a plurality of eyes having the same patterns (in Fig. 5, three substantially square eyes). By contrast, the other ion generating device 2 has, though not illustrated, the discharge electrode 4 formed in the shape of a grid with a plurality of eyes having dissimilar patterns. Dissimilar patterns are formed, for example, by forming each eye in different size or by forming pointed-end portions 24 at different positions from one eye to another.

With these two ion generating devices 2, the ion balance was checked, and it was found that, as expected, the one with a grid having a uniform pattern as shown in Fig. 5 yielded a better ion balance, meaning that it generated approximately equal numbers of positive and negative ions. The reason is considered to be that, in this ion generating device 2, the grid that is formed to have a uniform pattern contributes to producing a uniform electric field between the discharge and induction electrodes 4 and 5, resulting in a proper balance between the numbers of positive and negative ions generated. Thus, from the viewpoint of a proper ion balance, it is preferable to use an ion generating device 2 with a grid having a uniform pattern rather than one with a grid having a non-uniform pattern.

### Example 6

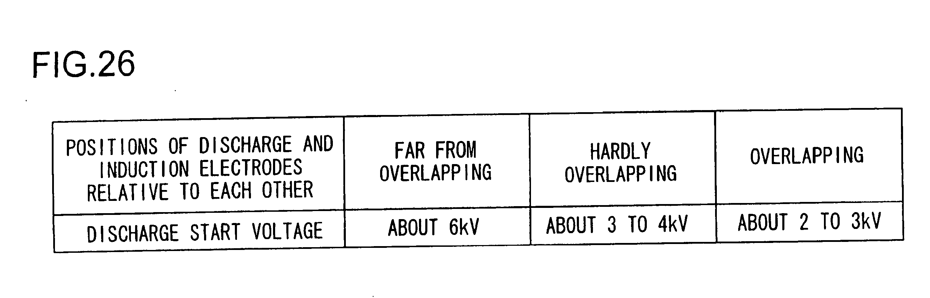

In this example, how the discharge start voltage varied with the positions of the discharge and induction electrodes 4 and 5 relative to each other was investigated. The results are shown in Fig. 26.

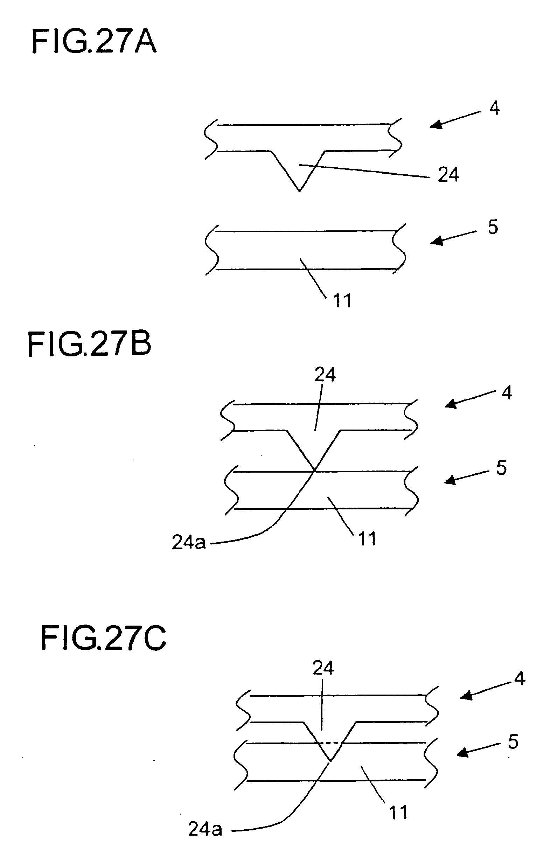

In Fig. 26, the discharge and induction electrodes 4 and 5 being "far from overlapping" denotes a state where, as shown in Fig. 27A, the pointed-end portions 24 of the discharge electrode 4 do not at all overlap the elongate portions 11 of the induction electrode 5. The discharge and induction electrodes 4 and 5 being "hardly overlapping" denotes a state where, as shown in Fig. 27B, the pointed-end portions 24 of the discharge electrode 4 do not overlap the elongate portions 11 of the induction electrode 5, but, as seen in a plan view, they do make contact with each other. The discharge and induction electrodes 4 and 5 being "overlapping" denotes a state where, as shown in Fig. 27C, the tip portions 24a of the pointed-end portions 24 of the discharge electrode 4 lie right above the elongate portions 11 of the induction electrode 5 as seen in a plan view.

From the results shown in Fig. 16, it is understood that, as the positions of the discharge and induction electrodes 4 and 5 relative to each other vary from the state shown in Fig. 27A to that shown in Fig. 27C, the discharge start voltage gradually decreases. In the state where, as shown in Fig. 27C, the tip portions 24a of the pointed-end portions 24 of the discharge electrode 4 completely overlap the induction electrode 5, the discharge start voltage is lowest. The reason is considered to be that, in the state shown in Fig. 27C, the electric field readily concentrates between the pointed-end portions 24 of the discharge electrode 4 and the induction electrode 5, making it easy for electric discharge to occur between the electrodes even at a low voltage.

Thus, forming the pointed-end portions 24 of the discharge electrode 4 so that the tip portions 24a thereof overlap the induction electrode 5 helps to induce electric discharge stably while keeping the discharge start voltage low, surely leading to lower power consumption.

### Example 7

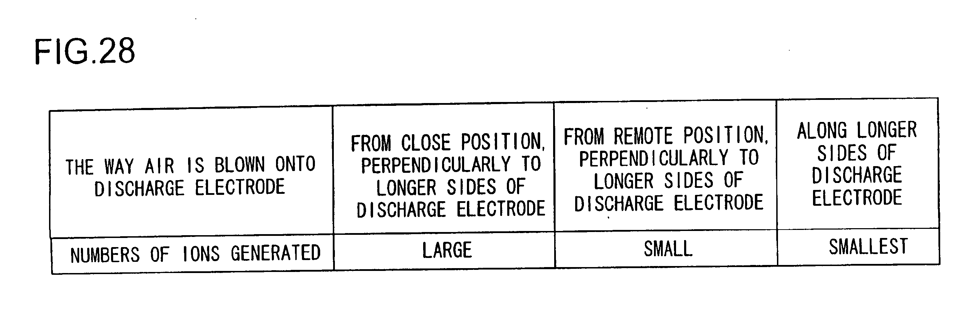

In this example, while the way air was blown onto the discharge electrode 4 (the stream of air) was varied, how the numbers of ions generated varied was investigated. The results are shown in Fig. 28. Here, it is assumed that the discharge electrode 4 is formed to have a line-symmetric pattern as shown in Fig. 5.

As shown in Fig. 28, as the way air is blown onto the discharge electrode 4 varies, the numbers of ions released into air vary. Though not illustrated, when the discharge electrode 4 was given a non-line-symmetric pattern, the numbers of ions generated varied more violently than when it was given a line-symmetric pattern.

The results indicate that, to minimize the variation of the numbers of ions generated under the influence of the stream of air, it is preferable that the discharge electrode 4 be so formed as to have a line-symmetric pattern.

In Examples 6 and 7, experiments were conducted with a discharge electrode 4 formed in the shape of a grid as shown in Fig. 5. Similar results were confirmed to be obtained also with a discharge electrode 4 formed in the shape of comb teeth as shown in Fig. 7 as with one formed in the shape of a grid.

### EMBODIMENT 2

Another embodiment of the present invention will be described below with reference to the relevant drawings. Such members as are found also in Embodiment 1 will be identified with common reference numerals, and their explanations will not be repeated.

This embodiment deals with the relationship between the thickness of the coating layer 8 and the thickness of the discharge electrode 4 as observed in the ion generator 1 described in connection with Embodiment 1.

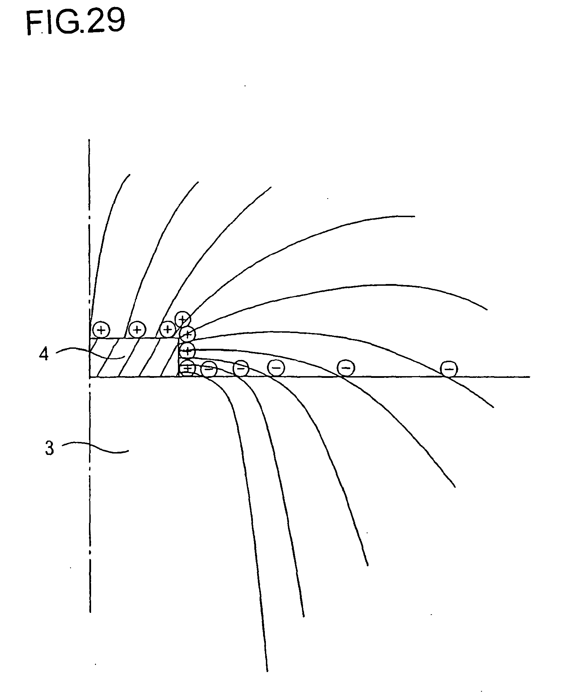

Fig. 29 shows the lines of electric force running from the discharge electrode 4 to the induction electrode 5 with a positive voltage applied to the discharge electrode 4 and a negative voltage to the induction electrode 5 (see Fig. 4) as observed in a case where the coating layer8 is not formed on the dielectric member 3. Here, the discharge electrode 4 is assumed to be 20 µm thick.

When the potential difference between the discharge and induction electrodes 4 and 5 induces corona discharge near the discharge electrode 4, as shown in Fig. 29, the potential on the surface of the dielectric member 3 polarizes along the lines of electric force to eventually become negative. Its intensity is higher the higher the dielectric constant of the dielectric member 3, and the lines of electric force are denser (the density of the lines of electric force is higher) than when no dielectric member 3 is present. As a result, the obtained electric field is such as if the induction electrode 5 were closer to the discharge electrode 4.

This phenomenon results from the lines of electric force (with a lower density than in Fig. 29) obtained when no dielectric member 3 is present, and thus, the higher the density of the lines of electric force in that case, the higher their density when the dielectric member 3 is present. The density of the lines of electric force indicates the strength of the electric field.

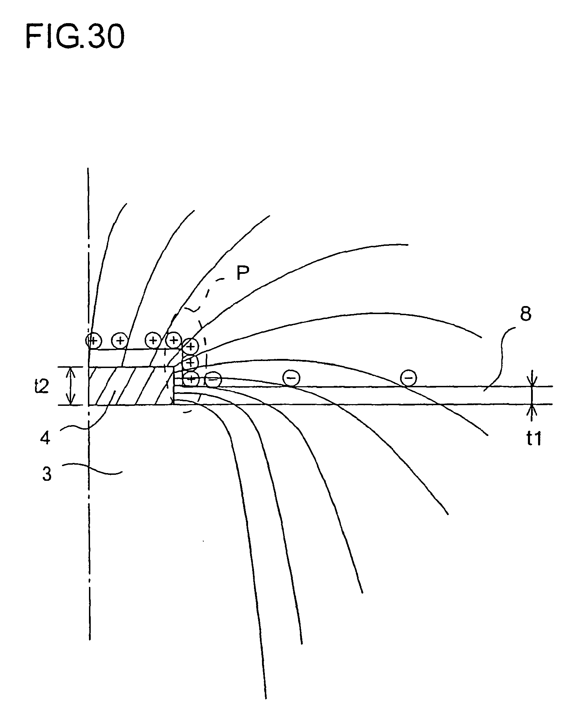

With the above assumptions in mind, next, a description will be given of the dielectric polarization that occurs, in a case where a coating layer 8 is formed on the dielectric member 3, on the coating layer 8. Figs. 30 to 32 show the lines of electric force running from the discharge electrode 4 to the induction electrode 5 with a positive voltage applied to the discharge electrode 4 and a negative voltage to the induction electrode 5 as observed in a case where a coating layer 8 is formed on the dielectric member 3.

The difference among Figs. 30 to 32 is that the thickness t1 of the coating layer 8 as measured in an area on the dielectric member 3 where the discharge electrode 4 is not formed is 11 µm, 22 µm, and 50 µm in Figs. 30, 31, and 32, respectively, while the thickness t2 of the discharge electrode 4 remains 20 µm in all these diagrams. Here, it is assumed that "parts of the coating layer 8 located in an area on the dielectric member 3 where the discharge electrode 4 is not formed" exclude those parts of the coating layer 8 that cover the edge surfaces of the discharge electrode 4.

When the potential difference between the discharge and induction electrodes 4 and 5 induces corona discharge near the discharge electrode 4, dielectric polarization occurs on the surface of the coating layer 8. At this time, as shown in Figs. 30 to 32, the surface potential of those parts of the coating layer 8 located near the discharge electrode (for example, those parts of the coating layer 8 located on the discharge electrode 4) polarizes to the same polarity as the discharge electrode 4 to become positive. On the other hand, the surface potential of those parts of the coating layer 8 located away from the discharge electrode (for example, those parts of the coating layer 8 located in an area on the dielectric member 3 where the discharge electrode 4 is not formed) polarizes to the opposite polarity to the discharge electrode 4 to become negative.

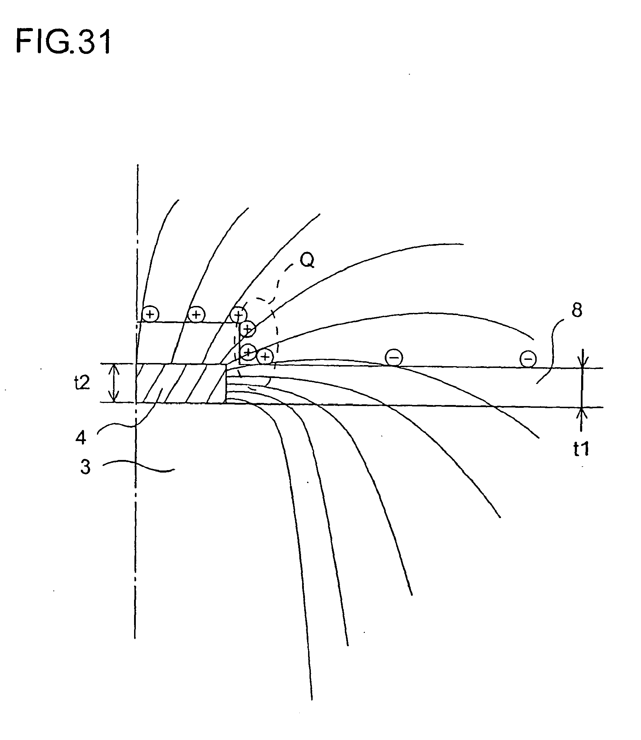

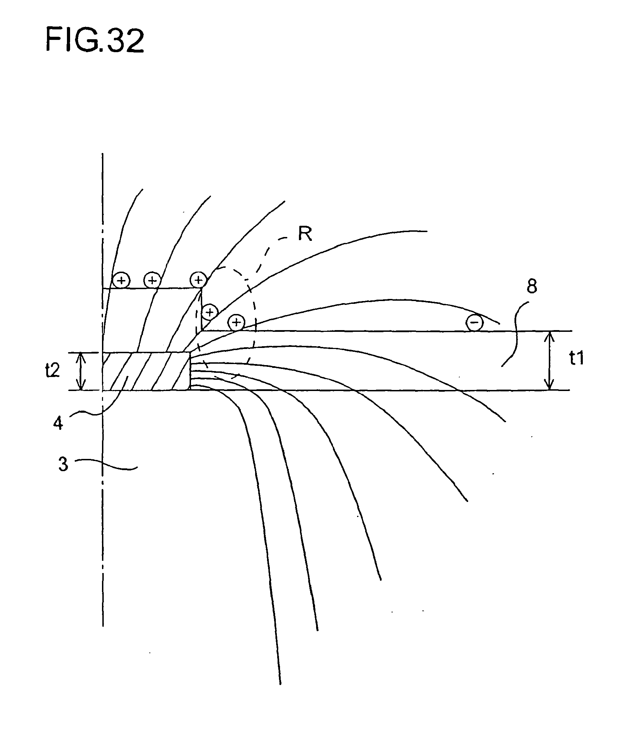

At this time, the lines of electric force running between the discharge and induction electrodes 4 and 5 are denser the closer they come to the discharge electrode 4, and the potential on the surface of the coating layer 8 starts polarizing at positions where the lines of electric force are denser when t1 is smaller than t2 than when t1 is substantially equal to t2 (as shown in Fig. 31) or greater than t2 (as shown in Fig. 32).

That is, in Figs. 30 to 32, let the spots where the electric field is strongest (the lines of electric force are densest) be P, Q, and R, respectively, then the intensity of the electric field at these spots are such that P > Q > R in decreasing order of electric field strength. This shows that, in a structure like the one under discussion, where the coating layer 8 is provided, if t1 becomes greater than t2, the strength of the electric field on the surface of the coating layer 8 becomes extremely weak.

With sample products having the coating layer 8 formed with varying thicknesses as in Figs. 20 to 32, the relationship between the output voltage (the voltage between the discharge and induction electrodes 4 and 5, also referred to as the line-to-line voltage) and the numbers of positive and negative ions generated was investigated. The results are shown in Table 1. Here, the input voltage (the voltage supplied to the voltage feeding circuit 9) was varied in the range from 55 V to 100 V, the output voltage was varied in the range from 2.62 kV to 4.2 kV, and the frequency of this voltage was about 40 kHz.

**TABLE 1**

| Coating Layer Thickness (µm) | Output Voltage (kV) | Positive Ions (× 10⁴ /cc) | Negative Ions (× 10⁴ /cc) |
|---|---|---|---|
| 11 | 2.62 | 6.0 | 6.5 |
| | 3.0 | 17.0 | 18.5 |
| | 3.4 | 24.0 | 26.0 |
| 22 | 2.69 | 3.5 | 4.5 |
| | 3.0 | 10 | 13 |
| | 3.4 | 14 | 18 |
| 50 | 3.84 | 2.5 | 4.0 |
| | 4.2 | 10 | 11 |

These results show that making t1 smaller than t2 helps to generate positive and negative ions in more equal numbers and thereby to keep a proper ion balance. This is possible because making t1 smaller than t2 permits the electric field to concentrate more (increases the intensity of the electric field) near the discharge electrode 4, permitting stable generation of positive and negative ions near the discharge electrode 4.

Moreover, as a result of making t1 smaller than t2 increasing the electric field strength near the discharge electrode 4, even when the potential difference between the discharge and induction electrodes 4 and 5 (the line-to-line voltage) is reduced, it is still possible to generate the desired numbers of positive and negative ions near the discharge electrode 4. This helps to reduce the power consumed by the ion generating device, and to reduce the discharge noise produced by the ion generating device. Thus, it is possible to reduce the amount of ozone, hazardous to the human heath, produced during electric discharge, making the ion generating device highly safe to use

An ion generating device according to the present invention may be so structured that the thickness of the protective layer in an area where the discharge electrode is not formed is smaller than the thickness of the protective layer on the discharge electrode.

The present invention is applicable also to ion generating devices that generate positive or negative ions alone.

### Industrial applicability

An ion generating device, a method for manufacturing an ion generating device, an ion generator, and an electric apparatus according to the present invention are useful in killing or removing airborne bacteria present in air or removing hazardous substances present in air.

## Claims

1. An ion generating device (2) comprising:
a discharge electrode (4) formed on a surface of a dielectric member (3) and an induction electrode (5) formed inside the dielectric member (3) and arranged so as to face the discharge electrode (4),
the ion generating device (2) generating ions by inducing electric discharge between the electrodes (4, 5),
**characterized in that** the induction electrode (5) is formed in a U shape within a plane facing the discharge electrode (4), two elongate portions forming the U shape of the induction electrode(5) are located so as to face each other inside a contour of the discharge electrode (4),
the discharge electrode (4) is formed in a shape of a grid, and
in each cyc of the grid, pointed-end portions (24) are formed so as to project from the grid into the eye and thereby overlap the elongate portions of the induction electrode (5) only from outermost sides thereof.

2. The ion generating device (2) of claim 1,
wherein a plurality of eyes of the grid have an identical pattern.

3. The ion generating device (2) of claim 1,
wherein the discharge electrode(4) is so formed as to have a line-symmetric pattern.

4. An ion generator (1) comprising
the ion generating device (2) of claim 1 and
voltage feeding means (9) for applying a voltage to at least one of the discharge (4) and induction (5) electrodes.

5. An electric apparatus comprising
the ion generator of claim 4 and
releasing means (42, 43, 44) for releasing the ions generating by the ion generator (1) into air.

## Patentansprüche

1. Ionenerzeugungseinrichtung (2), mit:
einer Entladungselektrode (4), die auf einer Oberfläche eines dielektrischen Elements (3) ausgebildet ist, und mit einer Induktionselektrode (5), die im Inneren des dielektrischen Elements (3) ausgebildet ist und die so angeordnet ist, dass sie der Entladungselektrode (4) gegenüber steht,
wobei die Ionenerzeugungseinrichtung (2) Ionen durch Induzieren elektrischer Entladung zwischen den Elektroden (4, 5) erzeugt,
**dadurch gekennzeichnet,**
**dass** die Induktionselektrode (5) in einer U-Form innerhalb einer Ebene ausgebildet ist, die der Entladungselektrode (4) gegenüberliegt, wobei zwei längliche Bereiche die U-Form der Induktionselektrode (5) bilden und so angeordnet sind, dass sie einander innerhalb einer Kontur der Entladungselektrode (4) gegenüberstehen,
wobei die Entladungselektrode (4) in Form eines Gitters bzw. Netzes ausgebildet ist und
wobei in jeder Masche des Gitters bzw. Netzes spitze Endbereiche (24) ausgebildet sind, um vom Gitter bzw. Netz in die Masche hervorzustehen und **dadurch** mit den länglichen Bereichen der Induktionselektrode (5) ausschließlich von den äußersten Seiten davon zu überlappen.

2. Ionenerzeugungseinrichtung (2) nach Anspruch 1,
wobei eine Mehrzahl von Maschen des Gitters bzw. Netzes eine identische Anordnung besitzt.

3. Ionenerzeugungseinrichtung (2) nach Anspruch 1,
wobei die Entladungselektrode (4) derart ausgebildet ist, dass sie eine liniensymmetrische Anordnung besitzt.

4. Ionenerzeuger (1), mit:
der Ionenerzeugungseinrichtung (2) nach Anspruch 1 und
einer Spannungszuführeinrichtung (9) zum Anlegen einer Spannung an mindestens einer der Entladungs- (4) und Induktionselektroden (5).

5. Elektrische Vorrichtung, mit:
dem Ionenerzeuger nach Anspruch 4 und
einer Abgabeeinrichtung (42, 43, 44) zum Abgeben der durch den Ionenerzeuger (1) erzeugten Ionen in Luft.

## Revendications

1. Dispositif générateur d'ions (2) comprenant :
une électrode de décharge (4) formée sur une surface d'un élément diélectrique (3) et une électrode d'induction (5) formée à l'intérieur de l'élément diélectrique (3) et disposée de façon à être tournée vers l'électrode de décharge (4),
le dispositif générateur d'ions (2) générant des ions par induction d'une décharge électrique entre les électrodes (4, 5),
**caractérisé en ce que** l'électrode d'induction (5) est réalisée en forme en U dans un plan en regard de l'électrode de décharge (4), deux parties allongées constituant la forme en U de l'électrode d'induction (5) sont situées de façon à être en regard l'une de l'autre à l'intérieur d'un contour de l'électrode de décharge (4),
l'électrode de décharge (4) est réalisée sous la forme d'une grille, et
dans chaque oeil de la grille, des parties à extrémités en pointe (24) sont formées de manière à faire saillie à partir de la grille dans l'oeil et ainsi chevaucher les parties allongées de l'électrode d'induction (5) à partir de ses côtés les plus à l'extérieur uniquement.

2. Dispositif générateur d'ions (2) selon la revendication 1,
dans lequel une pluralité d'yeux de la grille présente un motif identique.

3. Dispositif générateur d'ions (2) selon la revendication 1,
dans lequel l'électrode de décharge (4) est formée de manière à présenter un motif symétrique et linéaire.

4. Générateur d'ions (1) comprenant
le dispositif générateur d'ions (2) selon la revendication 1 et
des moyens d'alimentation en tension (9) pour appliquer une tension à l'électrode de décharge (4) et/ou à l'électrode d'induction (5).

5. Appareil électrique comprenant :
le générateur d'ions selon la revendication 4 et
des moyens de libération (42, 43, 44) pour libérer dans l'air les ions générés par le générateur d'ions (1).
